# EUROPEAN PATENT SPECIFICATION

(11) **EP 4 725 544 B1**
(45) Date of publication and mention of the grant of the patent: **05.08.2026**
(21) Application number: 25201840.3
(22) Date of filing: 12.09.2025
(51) Int. Cl.: A61N 1/362, A61N 1/372, A61N 1/375

(54) **PACEMAKER MEDIATED TACHYCARDIA DETECTION AND TERMINATION IN DUAL CHAMBER LEADLESS PACEMAKER SYSTEMS**
HERZSCHRITTMACHERVERMITTELTE TACHYKARDIEERKENNUNG UND BEENDIGUNG IN ZWEIKAMMER-HERZSCHRITTMACHERSYSTEMEN OHNE ELEKTRODEN
DÉTECTION ET TERMINAISON DE TACHYCARDIE INDUITE PAR UN STIMULATEUR CARDIAQUE DANS DES SYSTÈMES DE STIMULATEUR CARDIAQUE SANS FIL À DOUBLE CHAMBRE

(30) Priority: 08.10.2024 US 202463704670 P; 05.09.2025 US 202519321055
(43) Date of publication of application: 15.04.2026
(73) Proprietor: Pacesetter, Inc., Sylmar, CA 91342 (US)
(72) Inventor: Harrison, Taylor Clayton, Sylmar, CA 91342 (US)
(74) Representative: Barker Brettell Sweden AB

(56) References cited:
- US-A1- 2016 121 128
- US-B2- 10 987 518
- WEI ZILIANG ET AL: "A Time-Varying Equivalent Circuit Modeling and Measuring Approach for Intracardiac Communication in Leadless Pacemakers", IEEE TRANSACTIONS ON BIOMEDICAL CIRCUITS AND SYSTEMS, IEEE, US, vol. 18, no. 4, 31 January 2024 (2024-01-31), pages 872 - 884, XP011980564, ISSN: 1932-4545, [retrieved on 20240201], DOI: 10.1109/TBCAS.2024.3360997
- MULPURU SIVA K ET AL: "Cardiac Pacemakers: Function, Troubleshooting, and Management Part 1 of a 2-Part Series", JOURNAL OF THE AMERICAN COLLEGE OF CARDIOLOGY, ELSEVIER, AMSTERDAM, NL, vol. 69, no. 2, 9 January 2017 (2017-01-09), pages 189 - 210, XP029878995, ISSN: 0735-1097, DOI: 10.1016/J.JACC.2016.10.061

## Description

### TECHNICAL FIELD

The present invention generally relates to dual chamber leadless pacemaker systems, and methods for use therewith, and in particular to such systems and methods for detecting and terminating a pacemaker mediated tachycardia (PMT).

### BACKGROUND

A PMT, also referred to as endless loop tachycardia, or a pacemaker reentrant tachycardia, occurs when a pacemaker system paces a ventricle at an inappropriately fast rate for a sustained period of time. PMT occurs when a ventricular event occurs at a time, during which the connective tissue between the atrium and ventricle can transmit retrograde electrical signals from the ventricle to the atrium. The conduction of the ventricular signal to the atrium provides a spurious electrical signal in the atrium that is considered to be a natural atrial event by the pacemaker system. The pacemaker system senses the spurious retrograde atrial signal and then paces the ventricle at a programmed atrioventricular (AV) time period, also known as an AV interval (AVI) or an AV delay (AVD), following the retrograde atrial signal. The paced ventricular signal is conducted to the atrium and is again erroneously detected by the pacemaker system as a natural atrial event. The pacemaker system therefore continues to pace the ventricle at a relatively high rate defined by the sum of the programmed AVI and the retrograde conduction time between the ventricle and atrium. The high rate is sustained indefinitely by the pacemaker system because the retrograde conduction ensures that the pacemaker system detects what appears to be high rate atrial events and tracks these spurious atrial events by generating corresponding high rate ventricular paces. A PMT can be caused, e.g., by a retrograde conduction that follows a premature ventricular contractions (PVC), or by another event that causes AV synchrony to be dissociated.

Explained another way, ventricular events, which are conducted in a retrograde direction to the atria cause atrial depolarizations. The pacemaker system senses this retrograde atrial depolarization and then, after the appropriate AVI, delivers a stimulus to the ventricle. Thus, the pacemaker system provides the antegrade conduction pathway for a reentrant circuit and the intrinsic conduction system of the heart provides the retrograde pathway. A repetitive cycle of ventricular retrograde P-wave synchronized pacing can then ensue, which is the PMT.

There are existing PMT termination techniques for use with conventional dual chamber pacemaker systems that include a subcutaneous pacemaker housing, also referred to as can or case, and leads that include electrodes within the right atrium and the right ventricle. For example, in a conventional dual chamber pacemaker system, once a PMT is detected it can be terminated by extending the post ventricular atrial refractory period (PVARP) long enough such that the retrograde P-wave is not tracked and the reentrant circuit is broken. Another technique for terminating PMT is by restarting the AV cycle, and more specifically, by delivering an atrial pacing signal at a fixed time after the retrograde P-wave. However, such existing PMT termination techniques cannot be applied directly to a leadless dual chamber pacemaker system due to the additional complications associated with two physically-independent atrial and ventricular leadless pacemakers.

U.S. Patent Nos. 10,987,518 B2 and 11,648,407 B2 disclose an implantable system including an atrial leadless pacemaker (aLP) and a ventricular leadless pacemaker (vLP) that are configured to terminate a PMT. In U.S. Patent No. 10,987,518 B2, one of the aLP or the vLP detects a PMT and informs the other one. The aLP initiates a PMT PA interval that is shorter than a PA interval that the aLP would otherwise use for atrial pacing if a PMT was not detected. The vLP initiates a PMT PV interval that is longer than the PMT PA interval. If an intrinsic atrial or ventricular event is detected before the PMT PA interval or the PMT PV interval expires, then these intervals will be terminated, otherwise an atrial chamber will be paced if the PMT PA interval expires, and/or a ventricular chamber will be paced if the PMT PV interval expires. This should have the effect of terminating the PMT. In U.S. Patent No. 11,648,407 B2, in response to the aLP detecting a PMT, the aLP initiates a PMT PA interval, but the aLP does not inform the vLP, via an implant-to-implant (i2i) communication, of an atrial sensed event that caused the PMT to be detected, thereby preventing the vLP from initiating a PV interval during the PMT PA interval. The aLP selectively terminates the PMT PA interval. Additionally, the aLP informs the vLP, via an i2i communication, of an intrinsic atrial event being detected during the PMT PA interval, or of an atrial paced event being performed in response to the PMT PA interval expiring without an intrinsic atrial event being detected during the PMT PA interval.

There is still a need for detection and termination of PMTs in dual chamber leadless pacemaker systems, and in particular during conditions when the i2i communication between the aLP and the vLP is compromised or lost.

Ziliang et al., A Time-Varying Equivalent Circuit Modeling and Measuring Approach for Intracardiac Communication in Leadless Pacemakers, IEEE Transactions on Biomedical Circuits and Systems, 18(4): 872-884 (2024) developed and tested a quasi-static and a continuous time-varying equivalent circuit model of an intracardiac channel for multi-chamber leadless cardiac pacemakers. It was concluded that the proposed circuit model served as a powerful tool for addressing time-varying challenges and simplifying *in vivo* or *ex vivo* experiments.

### SUMMARY

It is a general objective to provide a dual chamber leadless pacemaker system capable of detecting and terminating PMT in conditions when the i2i communication between the aLP and the vLP is compromised or lost.

This and other objectives are met by embodiments as disclosed herein.

An embodiment relates to a dual chamber leadless pacemaker system comprising an aLP configured to be implanted in or on an atrial cardiac chamber and comprising at least two electrodes arranged to sense an intrinsic atrial event, pace the atrial cardiac chamber and perform i2i communication, and a vLP configured to be implanted in or on a ventricular cardiac chamber and comprising at least two electrodes arranged to sense an intrinsic ventricular event, pace the ventricular cardiac chamber and perform i2i communication. The vLP is configured to monitor for a PMT in response to reception of an i2i communication from the aLP indicating compromised i2i communication from the vLP to the aLP. The vLP is also configured to, following detection of a PMT, initiate a PMT PV interval in response to reception of an i2i communication from the aLP indicating an atrial sensed event. The PMT PV interval is shorter than a PV interval that the vLP would otherwise use for ventricular pacing if the PMT was not detected. The vLP is further configured to deliver a ventricular pacing pulse to the ventricular cardiac chamber in response to the PMT PV interval expiring without an intrinsic ventricular event being detected during the PMT PV interval.

A further embodiment relates to a dual chamber leadless pacemaker system comprising an aLP configured to be implanted in or on an atrial cardiac chamber and comprising at least two electrodes arranged to sense an intrinsic atrial event, pace the atrial cardiac chamber and perform i2i communication, and a vLP configured to be implanted in or on a ventricular cardiac chamber and comprising at least two electrodes arranged to sense an intrinsic ventricular event, pace the ventricular cardiac chamber and perform i2i communication. The aLP is configured to monitor for a PMT based on intrinsic atrial events detected by the aLP and i2i communications from the vLP indicating ventricular paced (VP) events. The aLP is also configured to, in response to detection of compromised i2i communication from the vLP to the aLP, transmit an i2i communication to the vLP indicating compromised i2i communication from the vLP to the aLP. The aLP is further configured to interrupt its monitoring for PMT until i2i communication from the vLP to the aLP is functional. The vLP is configured to, in response to reception of the i2i communication from the aLP indicating compromised i2i communication from the vLP to the aLP, monitor for a PMT based on ventricular paced events caused by the vLP and i2i communications from the aLP indicating AS events.

The dual chamber leadless pacemaker systems of the present embodiments enable PMT monitoring, detection and termination also when the i2i communication from the vLP to the aLP is compromised. As a consequence, PMT monitoring and termination can be performed by the pacemaker systems not only when the LPs are able to bidirectionally communicate successfully with each other but also when only unidirectional communication is possible.

This summary is not intended to be a complete description of the embodiments of the present technology. Other features and advantages of the embodiments of the present technology will appear from the following description in which the preferred embodiments have been set forth in detail, in conjunction with the accompanying drawings and claims.

### BRIEF DESCRIPTION OF THE DRAWINGS

The embodiments, together with further objects and advantages thereof, may best be understood by referring to the following description taken together with the accompanying drawings, in which:
Fig. 1 illustrates a dual chamber leadless pacemaker system formed in accordance with certain embodiments described herein as implanted in a heart.
Fig. 2 is a block diagram of a leadless pacemaker (LP) in accordance with certain embodiments herein.
Fig. 3 illustrates an LP in accordance with certain embodiments herein.
Fig. 4 is a timing diagram demonstrating one embodiment of implant to implant (i2i) communication for a paced event.
Fig. 5 is a timing diagram demonstrating one embodiment of i2i communication for a sensed event.
Fig. 6 is a high level flow chart that summarizes operations of an aLP of the dual chamber leadless pacemaker system according to various embodiments.
Fig. 7 is a flow chart illustrating various embodiments of triggering a PMT response by the aLP.
Fig. 8 is a high level flow chart that summarizes operations of a vLP of the dual chamber leadless pacemaker system according to various embodiments.
Fig. 9 is a flow chart illustrating various embodiments of triggering a PMT response by the vLP.
Fig. 10 is a timing diagram illustrating operations of an aLP and a vLP in connection with detection of compromised V2A i2i communication.
Fig. 11 is a timing diagram illustrating operations of an aLP and a vLP in connection with detection of a PMT by the vLP and with compromised V2A i2i communication.
Fig. 12 is a timing diagram illustrating operations of an aLP and a vLP in connection with PMT termination by the vLP according to an embodiment.
Fig. 13 is a timing diagram illustrating operations of an aLP and a vLP in connection with PMT termination by the vLP according to an embodiment.
Fig. 14 is a timing diagram illustrating operations of an aLP and a vLP in connection with PMT termination by the vLP according to an embodiment.
Fig. 15 is a flow chart of operations of a vLP of the dual chamber leadless pacemaker system according to an embodiment.

### DETAILED DESCRIPTION

The present invention generally relates to dual chamber leadless pacemaker systems, and in particular to such systems for detecting and terminating a pacemaker mediated tachycardia (PMT).

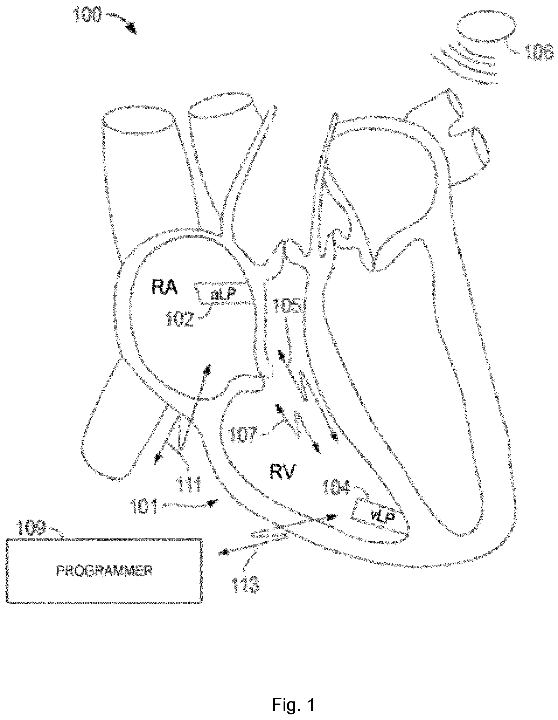

However, before providing addition details of the specific embodiments of the present technology, an exemplary dual chamber leadless pacemaker system, in which embodiments of the present technology can be used will first be described with reference to Figs. 1 to 3. More specifically, Figs. 1 to 3 will be used to describe an exemplary dual chamber leadless pacemaker system, wherein pacing and sensing operations can be performed by multiple implantable medical devices (IMDs), including an atrial leadless pacemaker (aLP) and a ventricular leadless pacemaker (vLP), and optionally one or more other IMDs, such as an implantable cardioverter-defibrillator (ICD), for instance, a subcutaneous-ICD, and/or one or more external, i.e., non-implantable devices, such as a programmer.

Fig. 1 illustrates a dual chamber leadless pacemaker system 100 formed in accordance with certain embodiments herein as implanted in a heart 101. The dual chamber leadless pacemaker system 100 comprises two or more leadless pacemakers (LPs) 102, 104 located in different chambers of the heart 101. A first LP is located in a right atrium (RA), also referred to as right atrial chamber, thereby denoted aLP 102 herein. A second LP is located in a right ventricle (RV), also referred to as right ventricular chamber, thereby denoted vLP 104. The aLP 102 and the vLP 104 communicate with one another to inform one another of various local physiologic activities, such as local intrinsic events, local paced events, and the like. The aLP 102 and the vLP 104 may be constructed in a similar manner, but operate differently based upon which cardiac chamber the LP 102, 104 is located. Instead of being implanted in the right atrium, the aLP 102 can be implanted on an exterior of the right atrium. Additionally, or alternatively, instead of being implanted in the right ventricle, the vLP 104 can be implanted on an exterior of the right ventricle.

In some embodiments, the aLP 102 and the vLP 104 communicate with one another, and optionally with another IMD, such as an ICD 106, and with an external device, such as a programmer 109, through wireless transceivers with connected telemetry coils and/or antenna, and/or by conductive communication through the same electrodes as used for sensing electrical events and/or delivery of pacing therapy. When conductive communication is maintained through the same electrodes as used for pacing, the dual chamber leadless pacemaker system 100 may omit an antenna or telemetry coil in one or both of the aLP 102 and the vLP 104. Hence, each of the aLP 102 and the vLP 104 preferably uses two or more electrodes located within, on, or within a few centimeters of the housing of the LP 102, 104, for pacing and sensing at the cardiac chamber and preferably also for bidirectional communication with one another, which is referred to implant-to-implant (i2i) communication herein, and optionally with the external device, such as a programmer 109, and/or another IMD, such as an ICD 106.

In some embodiments, the aLP 102 and the vLP 104 can be co-implanted with another IMD, such as an ICD 106. In such embodiment, the dual chamber leadless pacemaker system 100 also comprises an IMD, such as an ICD 106, in addition to the aLP 102 and the vLP 104.

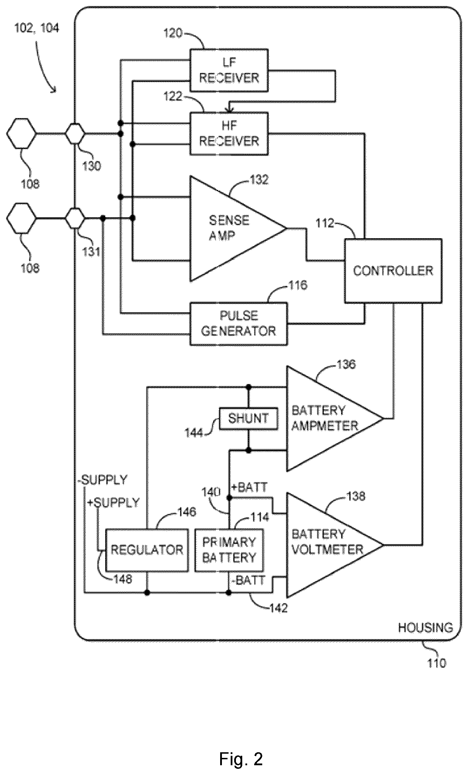

Fig. 2 is a block diagram showing exemplary circuits and electronics within the aLP 102 and the vLP 104, collectively referred to as LP 102, 104. In an embodiment, the LP 102, 104 includes a first receiver 120 and a second receiver 122 that collectively define separate first and second communication channels 105, 107, see Fig. 1, between the LPs 102, 104. Although first and second receivers 120, 122 are depicted in Fig. 2, in other embodiments, the LP 102, 104 only includes one of the first and second receivers 120, 122, or include additional receivers other than first and second receivers 120, 122. In Fig. 2, the first receiver 120 has been exemplified as a low frequency (LF) receiver 120 and the second receiver 122 has been exemplified as a high frequency (HF) receiver 122. As will be described in additional detail below, the pulse generator 116 of the LP 102, 104 can function as a transmitter that transmits i2i communication signals using the electrodes 108 located within, on, or within a few centimeters of the housing 110 of the LP 102, 104. Usage of the electrodes 108 for i2i communication enables the LP 102, 104 to perform antenna-less and telemetry coil-less communication, thereby relaxing the need for any such antenna or telemetry coil.

In accordance with certain embodiments, when one of the LPs 102, 104 senses an intrinsic event or delivers a paced event, the corresponding LP 102, 104 transmits an i2i communication, such as in the form of an implant event message, to the other LP 102, 104. For example, when the aLP 102 senses/paces an atrial event, the aLP 102 transmits an i2i communication including an event marker indicative of a nature of the event, for instance intrinsic/sensed atrial event or paced atrial event, to the vLP 104. When the vLP 104 senses/paces a ventricular event, the vLP 104 transmits an i2i communication including an event marker indicative of a nature of the event, for instance intrinsic/sensed ventricular event or paced ventricular event, to the aLP 102. In certain embodiments, the LP 102, 104 transmits an i2i communication to the other LP 102, 104 preceding the actual pacing pulse so that the other LP 102, 104 can blank its sense inputs in anticipation of that remote pacing pulse to prevent inappropriate crosstalk sensing.

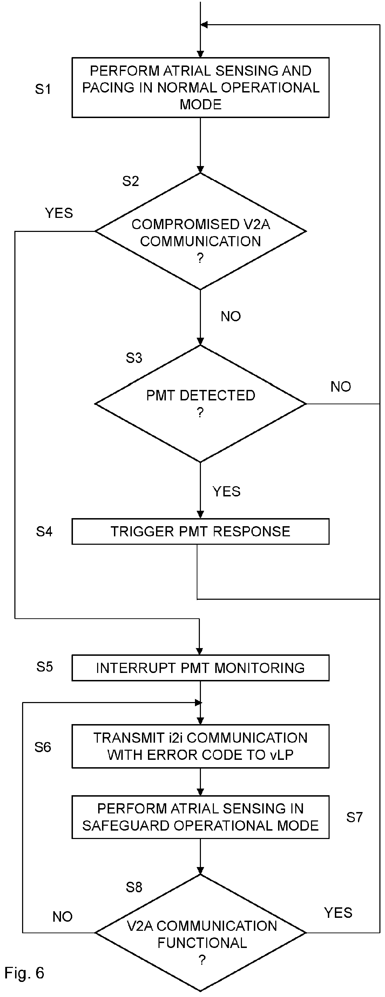

The LP 102, 104 as shown in Fig. 2 comprises a controller 112 and the above-mentioned pulse generator 116. The controller 112, also referred to as processor herein, can include, for instance a microprocessor or equivalent control circuitry, random access memory (RAM) and/or read-only memory (ROM), logic and timing circuitry, state machine circuitry, and input/output (I/O) circuitry, but is not limited thereto. The controller 112 can further include timing control circuitry to control the timing of the stimulation pulses, such as pacing rate atrioventricular (AV) interval (AVI), PV interval, atrial interconduction (A-A) interval, or ventricular interconduction (V-V) interval, etc. Such timing control circuitry may also be used for the timing of refractory periods, blanking intervals, noise detection windows, evoked response windows, alert intervals, marker channel timing, and so on. Such timing control circuitry can also be used to control the timing of the various intervals discussed below with reference to Figs. 6-15. The controller 112 can further include other dedicated circuitry and/or firmware and/or software components that assist in monitoring various conditions of the patient's heart and managing pacing therapies. The controller 112 and the pulse generator 116 may be configured to transmit i2i communications, via the electrodes 108, in a manner that does not inadvertently capture the heart in the chamber where LP 102, 104 is located, such as when the associated chamber is not in a refractory state. In addition, a LP 102, 104 that receives an i2i communication may enter an "event refractory" state, or event blanking state, following receipt of the i2i communication. The event refractory/blanking state may be set to extend for a determined period of time after receipt of an i2i communication in order to avoid the receiving LP 102, 104 from inadvertently sensing another signal as an i2i communication that might otherwise cause retriggering. For example, the receiving LP 102, 104 may detect a measurement pulse from another LP 102, 104 or an external device, such as a programmer 109.

In accordance with certain embodiments herein, the external device, such as programmer 109, may communicate over a programmer-to-LP channel, with the LP 102, 104 utilizing the same communication scheme. The external device, such as programmer 109, may listen to the i2i communications transmitted between LPs 102, 104 and synchronize programmer to implant communication such that external device, such as programmer 109, does not transmit communication signals 113 until after an i2i communication sequence is completed.

In accordance with certain embodiments, the LP 102, 104 may combine transmit operations with therapy. The transmitted event marker may be configured to have similar characteristics in amplitude and pulse width to a pacing pulse and the LP 102, 104 may use the energy in the i2i communications to help capture the heart. For example, a pacing pulse may normally be delivered with pacing parameters of 2.5 V amplitude, 500 ohm impedance, 60 bpm pacing rate, 0.4 ms pulse-width. The foregoing pacing parameters correspond to a current draw of about 1.9 pA. The same LP 102, 104 may implement an i2i communication utilizing event signaling parameters for amplitude, pulse-width, pulse rate, etc. that correspond to a current draw of approximately 0.5 pA for transmit.

The LP 102, 104 may combine the i2i communications with pacing pulses. For example, the LP 102, 104 may use a 50 µs wakeup transmit pulse having an amplitude of 2.5 V, which would draw 250 nC (nano Coulombs) for an electrode load of 500 ohm. The pulses of the event marker may be followed by an event message encoded with a sequence of short duration pulses, for example 16, 2 µs on/off bits, which would draw an additional 80 nC. The i2i communication pulse would then be followed by the remaining pulse-width needed to reach an equivalent charge of a nominal 0.4 ms pace pulse. In this case, the current necessary to transmit the event marker is essentially free as it was used to achieve the necessary pace capture anyhow. With this method, the savings in transmit current could be budgeted for the receiver or would allow for additional longevity.

When the LP 102, 104 senses an intrinsic event, it can send a qualitatively similar event pulse sequence, but indicative of a sensed event, without adding the pace pulse remainder. As the LP 102, 104 longevity calculations are designed based on the assumption that the LP 102, 104 will deliver pacing therapy 100% of the time, transmitting an intrinsic event marker to another LP 102, 104 will not impact the nominal calculated LP longevity.

In some embodiments, the LP 102, 104 comprises a hermetic housing 110 configured for placement on or attachment to the inside or outside of a cardiac chamber and at least two leadless electrodes 108 proximal to the housing 110 and configured for bidirectional communication with at least one other LP 102, 104 and optionally to another IMD within the body and/or an external device outside the body.

Fig. 2 depicts a LP 102, 104 and shows the LP's functional elements substantially enclosed in the hermetic housing 110. As mentioned above, the LP 102, 104 has at least two electrodes 108 located within, on, or near the housing 110, for delivering pacing pulses to and sensing electrical activity from the muscle of the cardiac chamber, and for bidirectional communication with at least one other device 102, 104, 106, 109 within or outside the body. The electrodes 108 may optionally be used for other sensing operations, such as sensing motion and/or for sensing temperature. Hermetic feedthroughs 130, 131 conduct electrode signals through the housing 110. The housing 110 contains a primary battery 114 to supply power for pacing, sensing, and communication. The housing 110 also contains sensing circuitry 132 for sensing cardiac activity from the electrodes 108, one or more receivers 120, 122 for receiving information from at least one other device via the electrodes 108, and the pulse generator 116 for generating pacing pulses for delivery via the electrodes 108 and also for transmitting information to at least one other device via the electrodes 108 via i2i communication. The housing 110 can further contain circuits for monitoring device health, for example a battery current monitor 136, such as in the form of a battery ampmeter, and a battery voltage monitor 138, such as in the form of a battery voltmeter, and can contain circuits for controlling operations in a predetermined manner.

Also shown in Fig. 2, the primary battery 114 has a positive terminal 140 and a negative terminal 142. Current from the positive terminal 140 of the primary battery 114 flows, in an embodiment, through a shunt 144 to a regulator circuit 146 to create a positive voltage supply 148 suitable for powering the remaining circuitry of the LP 102, 104. The shunt 144 enables the battery current monitor 136 to provide the controller 112 with an indication of battery current drain and indirectly of device health.

In some embodiments, the controller 112 in the LP 102, 104 can access signals on the electrodes 108 and can examine output pulse duration from another LP 102, 104 for usage as a signature for determining triggering information validity and, for a signature arriving within predetermined limits, activating delivery of a pacing pulse following a predetermined delay of zero or more milliseconds. The predetermined delay can be preset at manufacture, programmed via an external device, such as programmer 109, or determined by adaptive monitoring to facilitate recognition of the triggering signal and discriminating the triggering signal from noise.

In certain embodiments, the electrodes 108 of the LP 102, 104 can be used to sense an intracardiac electrocardiogram (IEGM), from which atrial and/or ventricular activity can be detected, e.g., by detecting R waves and/or P waves. Such an IEGM can also be used by an LP 102, 104 to time when i2i communication pulses should be generated, since the orientation of the LPs 102, 104 relative to one another can change throughout each cardiac cycle.

The electrodes 108 can be configured to communicate bidirectionally among the multiple LPs 102, 104 and optionally an IMD, such as an ICD 106, to coordinate pacing pulse delivery and optionally other therapeutic or diagnostic features using i2i communications that identify an event at an individual LP 102, 104 originating the message and an LP 102, 104 receiving the i2i communication react as directed by the message depending on the origin of the i2i communication. An LP 102, 104 that receives the i2i communication reacts as directed by the event message depending on the i2i communication origin or location. In some embodiments or conditions, the two or more leadless electrodes 108 can be configured to communicate bidirectionally among the LPs 102, 104, and optionally the IMD, and transmit data including designated codes for events detected or created by an individual LP 102, 104. Individual LPs 102, 104 can be configured to issue a unique code corresponding to an event type and a location of the sending LP 102, 104.

In some embodiments, an individual LP 102, 104 can be configured to deliver a pacing pulse with an event message or marker encoded therein, with a code assigned according to LP location and configured to transmit an i2i communication to one or more other LPs 102, 104 via the event message or marker coded pacing pulse. The LP 102, 104 or IMD 106 receiving the i2i communication are adapted to respond to the i2i communication in a predetermined manner depending on type and location of the event.

For instance, information communicated on the incoming channel can include an event message or marker from another LP 102, 104 signifying that the other LP 102, 104 has sensed an intrinsic cardiac activity or has delivered a pacing pulse, and identifies the location of the other LP. Similarly, information communicated on the outgoing channel can also include an event message or marker to another LP 102, 104, or to the IMD 106, that the sending LP 102, 104 has sensed an intrinsic cardiac activity or has delivered a pacing pulse at the location of the sending LP 102, 104.

As shown in the illustrative embodiments of Fig. 1, the LPs 102, 104 can be configured for operation in a particular location and a particular functionality at manufacture and/or at programming by an external device, such as programmer 109. Bidirectional i2i communication among the multiple LPs 102, 104 can be arranged to communicate notification of a sensed intrinsic event or delivered pacing pulse event and encoding type and location of the event to another implanted LP 102, 104. The LP 102, 104 receiving the i2i communication decode the information and respond depending on location of the receiving LP 102, 104 and predetermined system functionality.

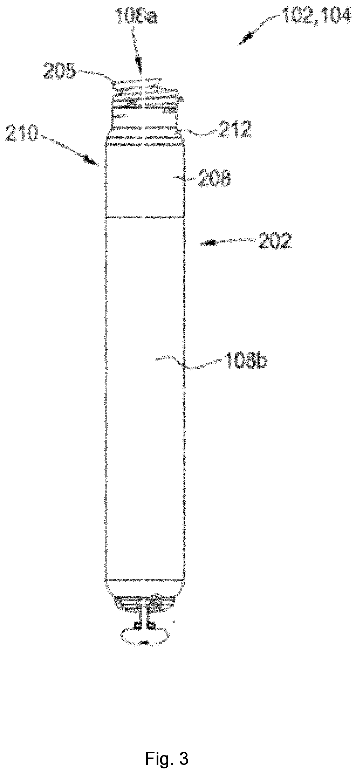

Fig. 3 shows an example of the LP 102, 104. The LP 102, 104 comprises a hermetic housing 202, such as the housing 110 in Fig. 2, with electrodes 108a, 108b disposed thereon. As shown, electrode 108a can be separated from but surrounded partially by a fixation mechanism 205, and the electrode 108b can be disposed on the housing 202, or a portion thereof. The fixation mechanism 205 can be a fixation helix, a plurality of hooks, barbs, or other attaching features configured to attach the LP 102, 104 to tissue, such as heart tissue. The electrodes 108a, 108b are examples of the electrodes 108 shown in and discussed above with reference to Fig. 2. One of the electrodes 108a, 108b, such as electrode 108a, can function as a cathode type electrode and the other of the electrodes 108a, 108b, such as electrode 108b, can function as an anode type electrode, or vice versa, when the electrodes 108a, 108b are used for delivering stimulation.

The housing 202 can also include an electronics compartment 210 within the housing 202 that contains the electronic components necessary for operation of the LP 102, 104, including, e.g., a pulse generator, receiver(s), a battery, and a controller for operation. The housing 202 can be adapted to be implanted on or in a human heart, and can be cylindrically shaped, rectangular, spherical, or any other appropriate shapes, for example.

The housing 202 can comprise a conductive, biocompatible, inert, and anodically safe material, such as titanium, 316L stainless steel, or other similar materials. The housing 202 can further comprise an insulator disposed on the conductive material to electrically separate electrodes 108a, 108b. The insulator can be an insulative coating on a portion of the housing between the electrodes 108a, 108b, and can comprise materials such as silicone, polyurethane, parylene, or another biocompatible electrical insulator commonly used for implantable medical devices. In the embodiment of Fig. 3, a single insulator 208 is disposed along the portion of the housing 202 between electrodes 108a, 108b. In some embodiments, the housing 202 itself can comprise an insulator instead of a conductor, such as an alumina ceramic or other similar materials, and the electrodes 108a, 108b can be disposed upon the housing 202.

As shown in Fig. 3, the LP 102, 104 can, optionally, include a header assembly 212 to isolate the electrodes 108a, 108b. The header assembly 212 can be made from polyether ether ketone (PEEK), tecothane or another biocompatible plastic, and can contain a ceramic to metal feedthrough, a glass to metal feedthrough, or other appropriate feedthrough insulator as known in the art.

The electrodes 108a, 108b can comprise pace/sense electrodes, or return electrodes. A low-polarization coating can be applied to the electrodes 108a, 108b, such as sintered platinum, platinum-iridium, iridium, iridium-oxide, titanium-nitride, carbon, or other materials commonly used to reduce polarization effects, for example. In Fig. 3, electrode 108a can be a pace/sense electrode and electrode 108b can be a return electrode. The electrode 108b can be a portion of the conductive housing 202 that does not include an insulator 208.

Several techniques and structures can be used for attaching the housing 202 to the interior or exterior wall of the heart. A helical fixation mechanism 205, can enable insertion of the device endocardially or epicardially through a guiding catheter. A torqueable catheter can be used to rotate the housing and force the fixation mechanism 205 into heart tissue, thus affixing the fixation mechanism 205, and also the electrode 108a in Fig. 3, into contact with stimulable tissue. Electrode 108b can serve as an indifferent electrode for sensing and pacing. The fixation mechanism 205 may be coated partially or in full for electrical insulation, and a steroid-eluting matrix may be included on or near the device to minimize fibrotic reaction, as is known in conventional pacing electrode-leads.

The LPs 102, 104 can utilize i2i communication through event messages to coordinate operation with one another in various manners. The terms i2i communication, i2i event messages, and i2i event markers are used interchangeably herein to refer to event related messages and operation related messages transmitted from an implanted device, such as an LP 102, 104, and directed to another implanted device, such as another LP 102, 104, although external devices, e.g., a programmer 109, may also receive i2i event messages. In certain embodiments, the LP 102 and the LP 104 operate as two independent leadless pacemakers maintaining beat-to-beat dual-chamber functionality via a "Master/Slave" operational configuration. The LP 102, 104 that is designated as the master device, e.g. vLP 104, may implement all or most dual-chamber diagnostic and therapy determination algorithms. For purposes of the following illustration, it is assumed that the vLP 104 is a "master" device, while the aLP 102 is a "slave" device. Alternatively, the aLP 102 may be designated as the master device, while the vLP 104 may be designated as the slave device. The master device orchestrates most or all decision-making and timing determinations (including, for example, rate-response changes).

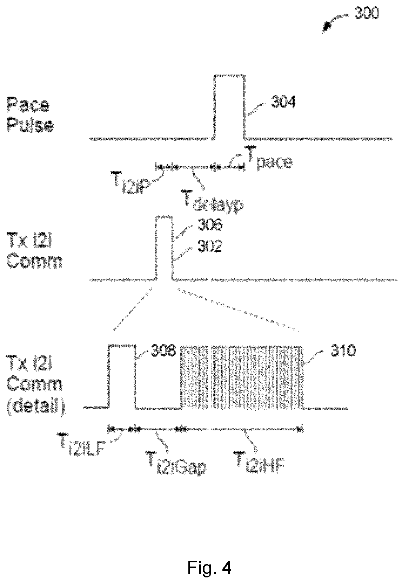

Fig. 4 is a timing diagram 300 demonstrating an example of an i2i communication for a paced event. The i2i communication may be transmitted, for example, from the aLP 102 to the vLP 104, or vice versa. As shown in Fig. 4, in this embodiment, an i2i transmission 302 is sent prior to delivery of a pace pulse 304 by the transmitting LP, e.g., aLP 102. This enables the receiving LP, e.g., vLP 104, to prepare for the remote delivery of the pace pulse. The i2i transmission 302 includes an envelope 306 that may include one or more individual pulses. For example, in this embodiment, the envelope 306 includes a low frequency pulse 308 followed by a high frequency pulse train 310. Low frequency pulse 308 lasts for a period Ti2iLF, and high frequency pulse train 310 lasts for a period Ti2iHF. The end of the low frequency pulse 308 and the beginning of high frequency pulse train 310 are separated by a gap period, Ti2iGap.

As shown in Fig. 4, the i2i transmission 302 lasts for a period Ti2iP, and the pace pulse 304 lasts for a period Tpace. The end of the i2i transmission 302 and the beginning of the pace pulse 304 are separated by a delay period, TdelayP. The delay period may be, for example, between approximately 0.0 and 10.0 milliseconds (ms), particularly between approximately 0.1 ms and 2.0 ms, and more particularly approximately 1.0 ms. The term approximately, as used herein, means +/- 10% of a specified value.

In an embodiment, an i2i transmission 302 comprises a low frequency (LF) component 308 and a high frequency (HF) component 310 as shown in Fig. 4. In such a case, the LF component 308 could be detected by the LF receiver 120 of the LP 102, 104. In an embodiment, the detection of the LF component 308 by the LF receiver 120 initiates power-up of the HF receiver 122 so that it is powered-up and can receive the HF component 310 of the i2i transmission 302.

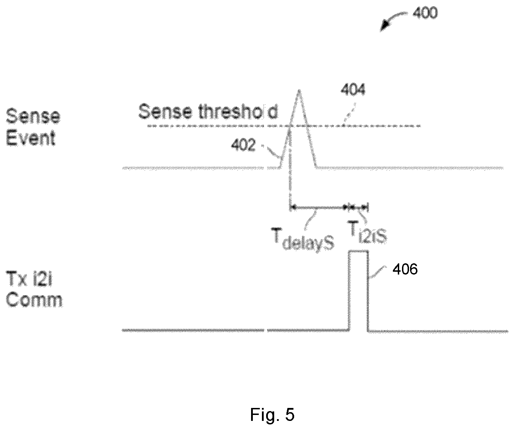

Fig. 5 is a timing diagram 400 demonstrating an example of an i2i communication for a sensed event. The i2i communication may be transmitted, for example, from the aLP 102 to the vLP 104, or vice versa. As shown in Fig. 5, in this embodiment, the transmitting LP, e.g., the aLP 102, detects the sensed event when a sensed intrinsic activation 402 crosses a sense threshold 404. A predetermined delay period, TdelayS, after the detection, the transmitting LP transmits an i2i transmission 406 that lasts a predetermined period Ti2iS. The delay period may be, for example, between approximately 0.0 and 10.0 milliseconds (ms), particularly between approximately 0.1 ms and 2.0 ms, and more particularly approximately 1.0 ms.

As with the i2i transmission 302, the i2i transmission 406 may include an envelope that may include one or more individual pulses. For example, similar to the envelope 306 in Fig. 4, the envelope of i2i transmission 406 may include a low frequency pulse followed by a high frequency pulse train.

In accordance with some embodiments, communication and synchronization between the aLP 102 and vLP 104 is implemented via conducted communication of markers/commands in the event messages (per i2i communication protocol). As explained above, conducted communication represents event messages transmitted from the sensing/pacing electrodes at frequencies outside the radio frequency (RF) or Wi-Fi frequency range. Alternatively and less preferred, the event messages may be conveyed over communication channels operating in the RF or Wi-Fi frequency range. The figures and corresponding description illustrate non-limiting examples of markers that may be transmitted in event messages. The figures and corresponding description also include the description of the markers and examples of results that occur in the LP that receives the event message. Table 1 represents exemplary event markers sent from the aLP 102 to the vLP 104, while Table 2 represents exemplary event markers sent from the vLP 104 to the aLP 102. In a master/slave configuration, AS event markers are sent from the aLP 102 each time that an atrial event is sensed outside of the post ventricular atrial blanking (PVAB) interval or some other alternatively-defined atrial blanking period. The AP event markers are sent from the aLP 102 each time that the aLP 102 delivers a pacing pulse in the atrium. The aLP 102 may restrict transmission of AS markers, whereby the aLP 102 transmits AS event markers when atrial events are sensed both outside of the PVAB interval and outside the post ventricular atrial refractory period (PVARP) or some other alternatively-defined atrial refractory period. Alternatively, the aLP 102 may not restrict transmission of AS event markers based on the PVARP, but instead transmit the AS event marker every time an atrial event is sensed.

**Table 1 - A2V makers/commands**

| Marker | Description | Result in vLP |
|---|---|---|
| AS | Notification of a sensed event in atrium (if not in PVAB or PVARP) | Initiate PV interval (if not in PVAB or PVARP) |
| AP | Notification of a paced event in atrium | Initiate PAVB |
| | | Initiate AV interval (if not in PVARP) |

As shown in Table 1, when an aLP 102 transmits an event message that includes an AS event marker (indicating that the aLP 102 sensed an intrinsic atrial event), the vLP 104 initiates a PV interval timer. If the aLP 102 transmits an AS event marker for all sensed events, then the vLP 104 would preferably first determine that a PVAB or PVARP interval is not active before initiating a PV interval timer. If, however, the aLP 102 transmits an AS event marker only when an intrinsic signal is sensed outside of a PVAB or PVARP interval, then the vLP 104 could initiate the PV interval timer upon receiving an AS event marker without first checking the PVAB or PVARP status. When the aLP 102 transmits an AP event marker (indicating that the aLP 102 delivered or is about to deliver a pace pulse to the atrium), the vLP 104 initiates a PVAB timer and an AV interval time, provided that a PVARP interval is not active. The vLP 104 may also blank its sense amplifiers to prevent possible crosstalk sensing of the remote pace pulse delivered by the aLP 102.

**Table 2 - V2A makers/commands**

| Marker | Description | Result in aLP |
|---|---|---|
| VS | Notification of a sensed event in ventricle | Initiate PVARP |
| VP | Notification of a paced event in ventricle | Initiate PVAB |
| | | Initiate PVARP |
| AP | Command to deliver immediate pace pulse in atrium | Deliver immediate pace pulse to atrium |

As shown in Table 2, when the vLP 104 senses a ventricular event, the vLP 104 transmits an event message including a VS event marker, in response to which the aLP 102 may initiate a PVARP interval timer. When the vLP 104 delivers or is about to deliver a pace pulse in the ventricle, the vLP 104 transmits VP event marker. When the aLP 102 receives the VP event marker, the aLP 102 initiates the PVAB interval timer and also the PVARP interval timer. The aLP 102 may also blank its sense amplifiers to prevent possible crosstalk sensing of the remote pace pulse delivered by the vLP 104. The vLP 104 may optionally transmit an event message containing an AP command marker to command the aLP 102 to deliver an immediate pacing pulse in the atrium upon receipt of the command without delay.

The foregoing event markers shown in Tables 1 and 2 are examples of a subset of markers that may be used to enable the aLP 102 and vLP 104 to maintain full dual chamber functionality. In an embodiment, the vLP 104 may perform all dual-chamber algorithms, while the aLP 102 may perform atrial-based hardware-related functions, such as PVAB, implemented locally within the aLP 102. In this embodiment, the aLP 102 is effectively treated as a remote 'wireless' atrial pace/sense electrode. In another embodiment, the vLP 102 may perform most but not all dual-chamber algorithms, while the aLP 102 may perform a subset of diagnostic and therapeutic algorithms. In an alternative embodiment, the vLP 104 and the aLP 102 may equally perform diagnostic and therapeutic algorithms. In certain embodiments, decision responsibilities may be partitioned separately to one of the aLP 102 or the vLP 104. In other embodiments, decision responsibilities may involve joint inputs and responsibilities.

As noted above, when using a pair of LPs 102, 104 to perform pacing and/or sensing operations in an atrial cardiac chamber, such as RA, and a ventricular cardiac chamber, such as RV, one of the challenges is terminating a PMT. PMT can result in any dual chamber pacemaker system capable of sensing and responding to atrial depolarizations when atrioventricular synchrony is dissociated, typically by a premature ventricular contract (PVC). Ventricular events are conducted in a retrograde direction to the atria that cause atrial depolarizations.

In a conventional dual chamber pacemaker system, once a PMT is detected it can be terminated by extending the PVARP long enough such that the retrograde P wave is not tracked and the reentrant circuit is broken. Another technique for terminating a PMT in a conventional dual chamber pacemaker system is by restarting the atrioventricular cycle, i.e., by delivering an atrial pacing output at a fixed time after the retrograde P-wave. However, such existing PMT termination techniques cannot be applied directly to a leadless dual chamber pacemaker system, such as the one summarized above with reference to Figs. 1-5, due to the additional complications associated with two physically-independent atrial and ventricular leadless pacing units, i.e., the aLP 102 and the vLP 104.

PMT termination techniques for dual chamber leadless pacemaker systems have been proposed in the art, such as in U.S. Patent Nos. 10,987,518 B2 and 11,648,407 B2. PMT detection and termination techniques for dual chamber leadless pacemaker systems are typically based on functional i2i communication between the aLP 102 and the vLP 104 and, in which one of the leadless pacemakers, typically the aLP 102, performs the actual PMT detection and termination but based on i2i communications from the other leadless pacemaker, typically the vLP 104. Such an approach thereby requires functional i2i communication between the aLP 102 and the vLP 104 and that both the aLP 102 and the vLP 104 are capable of performing sensing and pacing in accordance with a normal operational mode. Such a normal operational mode is, in an embodiment, a DDD or a DDD(R) pacing mode. DDD is a dual chamber and tracking mode that enables pacing in the atrium and the ventricle, sensing in the atrium and the ventricle and inhibiting or triggering delivery of pacing pulses to the atrium and to the ventricle. In the DDD pacing mode, an intrinsic P-wave and an intrinsic R-wave can inhibit delivery of atrial and ventricular pacing pulses, respectively, and intrinsic P-wave or atrial paced events can trigger a PV or AV delay. This mode fully adapts to intrinsic heart rhythm and mimics normal conduction as much as possible. The DDD pacing mode generally has four pacing patterns: ASVS (atrial sensed, ventricle sensed, used when the patient has good sinus node function and good AV node function), ASVP (atrial sensed, ventricular paced, used when the patient has a good sinus node function but poor AV node conduction), APVS (atrial paced, ventricular sensed, used when the patient has poor sinus node function but has intact AV node conduction) and APVP (atrial paced, ventricular paced, used when the patient has poor sinus and AV node function). DDD(R) is a DDD pacing mode with rate response or rate adaptive pacing. In such a DDD(R) pacing mode, the dual chamber leadless pacemaker system can sense motion, temperature, and/or minute ventilation changes indicative of to the patient activity and pace the heart at a required rate.

However, when the i2i communication between the aLP 102 and the vLP 104 is compromised, i.e., lost or unsuccessful, the dual chamber leadless pacemaker system reverts to a safeguard operational mode, sometimes referred to as back-up operational mode. In such a safeguard operational mode, the PMT detection and termination technique used during normal operational mode can no longer be used due to the comprised i2i communication and due to changed sensing and/or pacing abilities of the aLP 102 and/or the vLP 104 in the safeguard operational mode. An illustrative, but non-limiting, example of such a safeguard operational mode for a leadless dual chamber pacemaker system operating in the DDD or DDD(R) pacing mode as normal operational mode is a VDD or VDD(R) pacing mode. VDD is a dual chamber and tracking mode that enables pacing in the ventricle, sensing in the atrium and the ventricle and inhibiting or triggering delivery of pacing pulses to the ventricle. In the VDD pacing mode, an intrinsic R-wave can inhibit delivery of ventricular pacing pulses, and intrinsic P-wave can trigger a PV delay. In this mode, the dual chamber leadless pacemaker system does not pace the atrium, but an intrinsic atrial event can trigger the atrioventricular delay, resulting in P-wave tracking. Thus, in this mode, atrial pacing is withheld. VDD(R) is a VDD pacing mode with rate response or rate adaptive pacing.

The present invention enables detection and termination of a PMT also in situations where the i2i communication from the vLP 104 to the aLP 102 is compromised. The invention can thereby terminate PMTs also when the dual chamber leadless pacemaker system 100 operates in a safeguard operational mode, in which the aLP 102 does not deliver pacing pulses to the atrium. This means that the aLP 102 withholds atrial pacing.

Fig. 6 is a high level flow chart that summarizes operations of an aLP 102 of the dual chamber leadless pacemaker system 100 according to various embodiments.

Step S1 of Fig. 6 comprises the aLP 102 performing atrial sensing and pacing in accordance with the normal operational mode of the aLP 102, such as by collectively operating in a DDD or DDD(R) pacing mode with the vLP 104. Collectively operating in a pacing mode as used herein means that the aLP 102 and the vLP 104 operate together, i.e., collectively, to enable cardiac pacing in the given pacing mode. Such a normal operational mode may involve the aLP 102 sensing for intrinsic atrial events in the atrial cardiac chamber, and pacing the atrial cardiac chamber if a PA interval or AA interval expires without an intrinsic atrial event being detected during the PA interval or AA interval. In the terms "PA interval" and "AA interval", the "P" refers to an intrinsic atrial sensed event, which is also known as a P-wave, and the "A" refers to a paced atrial event caused by delivery of an A-pulse by the aLP 102.

When operating in the normal operational mode, the aLP 102 monitors for i2i communications transmitted by the vLP 104. Generally, the aLP 102 expects at least one such i2i communication per cardiac cycle and where such an i2i communication transmitted by the vLP 104 contains at least information indicating a ventricular sensed (VS) event, i.e., a sensed R-wave, or indicating a ventricular paced (VP) event. This is schematically indicated by step S2 where the aLP 102 determines whether the i2i communications from the vLP 104, denoted ventricular-to-atrial (V2A) i2i communication herein, are successfully received or whether the V2A i2i communication is compromised.

If the aLP 102 successfully receives i2i communications from the vLP 104, the flow continues to step S3. This step S3 involves monitoring for a PMT and determination of whether a PMT is detected. Thus, in an embodiment, the aLP 102 is responsible for PMT monitoring when the pacemaker system 100 is operating in the normal operational mode.

The aLP 102 can, in an embodiment, detect a PMT if, for a predefined number of consecutive cardiac cycles, the ventricular pacing rate exceeds a PMT detection rate and a ventricular paced event is, in each of these cardiac cycles, followed by an atrial sensed event. For instance, the aLP 102 could monitor VP-AS intervals for a predefined number of consecutive cycles when the ventricular pacing rate exceeds a PMT detection rate. From the perspective of the aLP 102, a VP-AS interval as the term is used herein represents a duration from reception of an i2i communication from the vLP 104 indicating a ventricular paced event to sensing an intrinsic atrial event by the aLP 102. Thus, if the aLP 102 detects the predefined number of consecutive VP-AS intervals and the ventricular pacing rate exceeds the PMT detection rate, the aLP 102 concludes that the criteria for PMT detection are met in step S3.

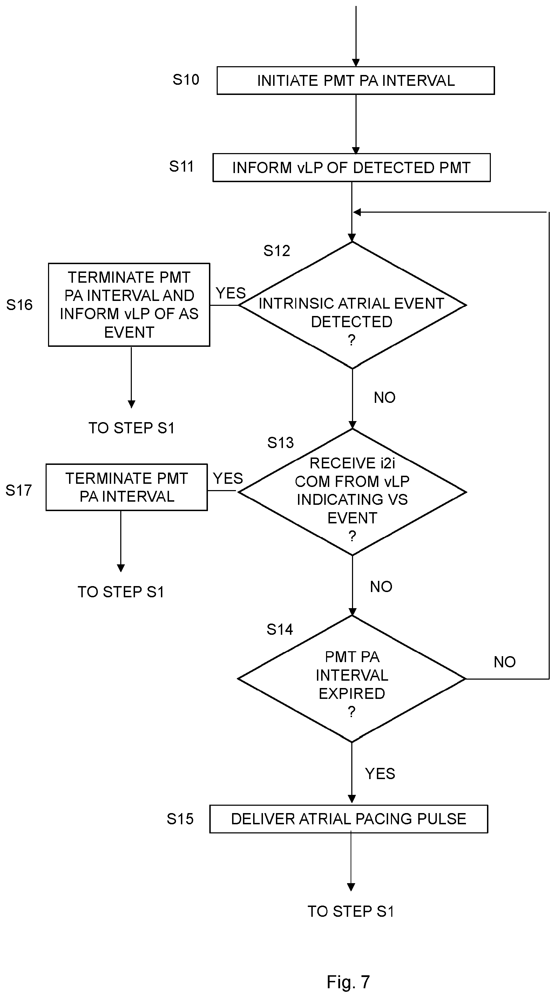

If no PMT is detected in step S3, the flow returns back to step S1. However, if a PMT is detected in step S3, the flow continues to step S4. At step S4 the aLP 102 triggers a PMT response. Fig. 7 is a flow chart illustrating various embodiments of triggering such a PMT response. In accordance with certain embodiments, the PMT response can involve initiating, in step S10, a PMT PA interval that is shorter than the PA interval that the aLP would otherwise use for atrial pacing if a PMT was not detected. The PA interval specifies when an atrial pacing pulse should be delivered by the aLP 102 to the atrial cardiac chamber following an intrinsic atrial sensed event, in response to the PA interval expiring without another intrinsic atrial event being detected during the PA interval, during a period that the PMT has not been detected. Where the PA interval that the aLP 102 would normally use for atrial pacing, if a PMT was not detected, is, e.g., 800 milliseconds (ms), then this means that an atrial pacing pulse (aka an A-pulse) will be delivered 800 ms after an intrinsic atrial sensed event (aka a P-wave), if the PA interval expires without an intrinsic atrial event being detected during the PA interval. This would provide for a heart rate (HR) of 75 beats per minute (bpm), since 60,000 ms per minute divided by 800 ms per beat equals 75. An exemplary range for the PA interval is from 600 to 1000 ms, which corresponds to a heart rate from 100 to 60 bpm. Lower and/or higher PA intervals are also possible, e.g., another exemplary range for the PA interval is from 400 to 2000 ms, which corresponds to a heart rate of 150 to 30 bmp. The PMT PA interval, which is initiated in step S10, is shorter than the PA interval that the aLP 102 would otherwise use for atrial pacing if a PMT was not detected. For example, the PMT PA interval can be 330 ms. An exemplary range for the PMT PA interval is from 250 ms to 399 ms, but is not limited thereto.

The PMT PA interval can be a programmed value, i.e., can be determined and programmed by a physician using the external device, such as programmer 106. Alternatively, the PMT PA interval could be a defined constant duration, i.e., non-programmable. In other embodiments, the PMT PA interval is equal to the PA interval minus a programmed or constant value, or the PMT PA interval can be a specified percentage of the of the PA interval.

Initiating the PMT PA interval in step S10 can involve the aLP 102 initiating a timer, which can be a dedicated PMT PA timer, or a same timer that is normally used for the PA interval can also be used for the PMT PA interval, depending upon the specific implementation.

The triggered PMT response can also involve the aLP 102 informing, in step S11, the vLP 104, via an i2i communication, of the detected PMT. For example, when the aLP 102 sends an i2i communication to the vLP 104, to inform the vLP 104 of an atrial sensed event, the same i2i communication, or a following i2i communication, can inform the vLP 104 of the PMT detected by the aLP 102.

Once the PMT PA interval is initiated as part of the PMT response triggered at step S4 in Fig. 6, the aLP 102 monitors for any intrinsic atrial event in step S12. If such an intrinsic atrial event is detected in step S12 prior to the PMT PA interval expiring, which is indicative of the PMT having terminated, the aLP 102 terminates the PMT PA interval in step S16, notifies the vLP 104 of the AS event via i2i communication, and the flow then returns to step S1. Correspondingly, if the aLP 102 receives an i2i communication from the vLP 104 in step 13 indicating an intrinsic ventricular event was sensed by the vLP 104 prior to the PMT PA interval expiring, which is indicative of the PMT having terminated, the aLP 102 will terminate the PMT PA interval in step S17 and the flow then returns to step S1. In these cases, the aLP 102 presumes that the PMT has been terminated. However, if the PMT PA interval expires in step S14 without the detection of any intrinsic atrial event by the aLP 102 or without any intrinsic ventricular event being detected by the vLP 104, then the aLP 102 delivers an atrial pacing pulse to the atrial cardiac chamber at the end of the PMT PA interval in an attempt to terminate the PMT in step S15. The aLP 102 also informs the vLP 104 of the atrial paced event via an i2i communication. The flow then returns to step S1. If the PMT PA interval has not expired in step S14, the method returns to step S12.

The above-described operations thereby involve PMT monitoring and termination when the dual chamber leadless pacemaker system 100 is operating in the normal operational mode and when the i2i communication between the aLP 102 and the vLP 104 is functional. However, if the aLP 102 detects that the V2A i2i communication is compromised in step S2, the flow instead continues to step S5 in Fig. 6.

The aLP 102 can determine that the V2A i2i communication is compromised according to various embodiments. For instance, the aLP 102 can determine that the V2A i2i communication is compromised if the aLP 102 has not received any i2i communication from the vLP 104 during a predefined number of cardiac cycles. This predefined number of cardiac cycles could be number equal to or larger than 1. As an example, the aLP 102 determines the V2A i2i communication is compromised on a cycle by cycle basis. In such an example, the aLP 102 determined that the V2A i2i communication is compromised if if does not receive a valid i2i communication from the vLP 104 from when it last sensed atrial activity or paced the atrium to when the next sensed or paced event occurs. In another embodiment, the aLP 102 listens for V2A i2i communications from the vLP 104 during a predefined period of time and if no such V2A i2i communication has been received from the vLP 104 during the predefined period of time the aLP 102 determines that the V2A i2i communication is compromised in step S2.

At step S5 the aLP 102 interrupts its PMT monitoring in step S6 as the PMT monitoring previously performed by the aLP 102 in the normal operational mode in step S3 requires functional V2A i2i communication. The vLP 104 will, as is further described herein, be responsible for PMT monitoring in response to reception of the i2i communication with the error code transmitted by the aLP 102 in step S6. This step S6, thus, involves the aLP 102 transmitting an i2i communication to the vLP 104 indicating that the V2A i2i communication is compromised, indicated as an i2i communication with an error code in Fig. 6.

The aLP 102 further starts operation in the safeguard operational mode in step S7. In an embodiment, this safeguard operational mode involves atrial sensing but not atrial pacing. Hence, an example of such a safeguard operational mode is when the aLP 102 and the vLP104 collectively operate in the VDD or the VDD(R) pacing mode. At step S8 the aLP 102 monitors for i2i communication from the vLP 104 to thereby determine whether the V2A i2i communication is still compromised or has become functional. In case of retained compromised V2A i2i communication, the flow continues to step S6 and the aLP 102 transmits an i2i communication to the vLP 102 with an error code to indicate that the V2A i2i communication is still compromises. The aLP 102 accordingly continues to operate in the safeguard operational mode in step S7. However, if the aLP 102 is able to successfully receive i2i communication from the vLP 104 the flow continues from step S8 to step S1 and the aLP 102 switches back to the normal operational mode.

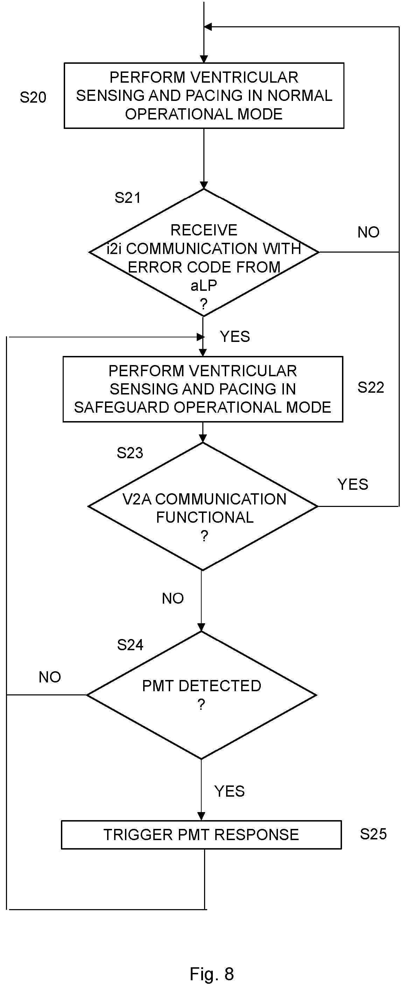

Fig. 8 is a high level flow chart that summarizes operations of a vLP 104 of the dual chamber leadless pacemaker system 100 according to various embodiments.

Step S20 of Fig. 8 comprises the vLP 104 performing ventricular sensing and pacing in accordance with the normal operational mode of the vLP 104, such as by collectively operating in a DDD or DDD(R) pacing mode together with the aLP 102. Such a normal operational mode may involve the vLP 104 sensing for intrinsic ventricular events in the ventricular cardiac chamber, and pacing the ventricular cardiac chamber if a PV interval or AV interval expires without an intrinsic ventricular event being detected during the PV interval or AV interval. In the terms "PV interval" and "AV interval", the "P" refers to an intrinsic atrial sensed event, which is also known as a P-wave, the "A" refers to a paced atrial event caused by delivery of an A-pulse by the aLP 102 and "V" refers to a paced ventricular event caused by delivery of a V-pulse by the vLP 104.

When operating in the normal operational mode the vLP 104 monitors for i2i communications transmitted by the aLP 102. Generally, the vLP 104 expects at least one such i2i communication per cardiac cycle and where such an i2i communication transmitted by the aLP 102 contains at least information indicating an atrial sensed (AS) event, i.e., a sensed P-wave, or indicating a atrial paced (AP) event. If the aLP 102 determines in step S2 in Fig. 6 that the V2A i2i communication is compromised the aLP 102 transmits an i2i communication with an error code or another form of indication of the compromised V2A i2i communication to the vLP 104 in step S5 in Fig. 6. This means that the vLP 104 investigates whether i2i communications received from the aLP 102 comprise any such error code or other form of indication of the compromised V2A i2i communication in step S11. If the received i2i communication does not contain any such error code or indication the flow continues to step S20 and the vLP 104 continues operating in the normal operation mode.

However, if the vLP 104 determines in step S21 that an i2i communication from the aLP 102 comprises an error code or another form of indication of the compromised V2A i2i communication the flow continues to step S22. The vLP 104 then switches to the safeguard operational mode. In an embodiment, this safeguard operational mode involves ventricular sensing and pacing. Hence, an example of such a safeguard operational mode is when the vLP 104 and the aLP102 collectively operate the VDD or the VDD(R) pacing mode. The flow continues to step S23 at which the vLP 104 monitors whether the V2A i2i communication is still compromised or functional. This monitoring is preferably performed based on i2i communications received from the aLP 102 and where such i2i communications contain the previously mentioned error code or other form of indication as long as the V2A i2i communication is compromised. Additionally, if the vLP 104 does not receive any i2i communication from the aLP 102 during a cardiac cycle, while the vLP 104 is operating in the safeguard operational mode, the vLP 104 can assume that the V2A i2i communication continues to be compromised.

If the vLP 104 determines in step S23 that the V2A i2i communication is once again functional, the flow continues to step S10 and the vLP 104 switches back to operating in the normal operational mode. However, if the V2A i2i communication is still compromised the vLP 104 assumes responsibility for PMT monitoring in step S24. This step S24 involves the vLP 104 monitoring for a PMT and determination of whether a PMT is detected. Thus, in an embodiment, the vLP 104 is responsible for PMT monitoring when the dual chamber leadless pacemaker system 100 is operating in the safeguard operational mode and the V2A communication is compromised.

The vLP 104 can, in an embodiment, detect a PMT if, for a predefined number of consecutive cardiac cycles, the ventricular pacing rate exceeds a PMT detection rate and a ventricular paced event is, in each of these cardiac cycles, followed by an atrial sensed event. For instance, the vLP 104 could monitor VP-AS intervals for a predefined number of consecutive cycles when then ventricular pacing rate exceeds a PMT detection rate. From the perspective of the vLP 104, a VP-AS interval as the term is used herein represents a duration from a ventricular paced event to reception of an i2i communication from the aLP 102 indicating an intrinsic atrial event sensed by the aLP 102. Thus, if the vLP 104 detects the predefined number of consecutive VP-AS intervals and the ventricular pacing rate exceeds the PMT detection rate, the vLP 104 concludes that the criteria for PMT detection are met in step S14.

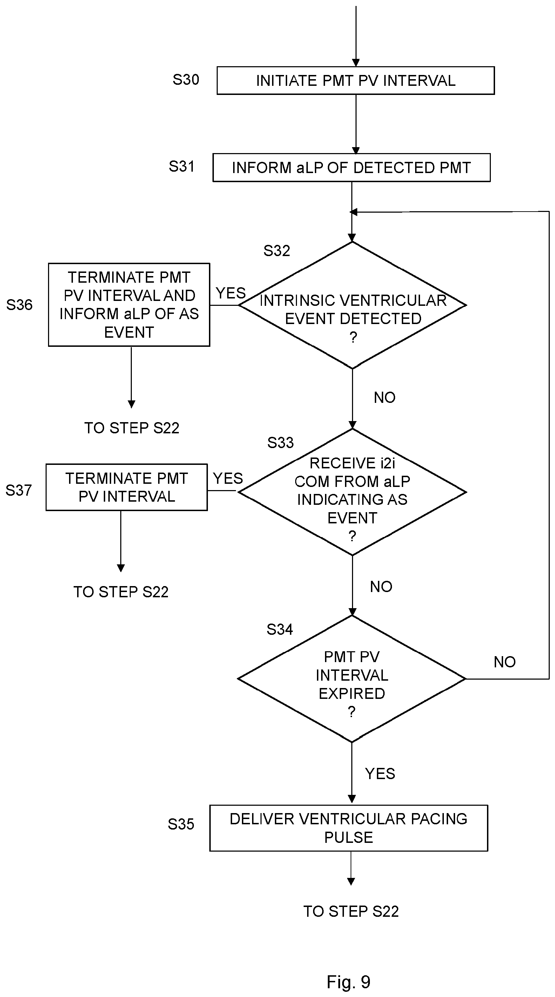

If no PMT is detected in step S24, the flow returns back to step S22. However, if a PMT is detected in step S24, the flow continues to step S25. At step S25 the vLP 104 triggers a PMT response. Fig. 9 is a flow chart illustrating various embodiments of triggering such a PMT response. In accordance with certain embodiments, the PMT response can involve initiating, in step S30, a PMT PV interval that is shorter than the PV interval that the vLP 104 would otherwise use for ventricular pacing if a PMT was not detected. The PV interval specifies when a ventricular pacing pulse should be delivered by the vLP 104 to the ventricular cardiac chamber following an atrial sensed event in response to the PV interval expiring without an intrinsic ventricular event being detected during the PV interval. Thus, the vLP 104 initiates the PV interval in response to reception of an i2i communication from the aLP 102 indicating an AS event. The vLP 104 then monitors for any intrinsic ventricular event, e.g., by monitoring for an R-wave (or alternatively, an entire QRS complex), during this PV interval and then delivers a pacing pulse to the ventricular cardiac chamber at the end of the PV interval if no such intrinsic ventricular event is detected by the vLP 104 during the PV interval. The PV interval as referred to herein is the interval used by the vLP 104 for ventricular pacing if a PMT is not detected. Correspondingly, PMT PV interval as used herein is the PV interval used by the vLP 104 for ventricular pacing if a PMT is detected. This means that PMT PV interval specifies when a ventricular pacing pulse should be delivered by the vLP 104 to the ventricular cardiac chamber following an atrial sensed event in response to the PMT PV interval expiring without an intrinsic ventricular event being detected during the PMT PV interval, during a period when the PMT has been detected.

According to an embodiment of the invention, the PMT PV interval is shorter than the PV interval that the vLP 104 would otherwise use for ventricular pacing if PMT was not detected. The shortening of the duration of the PMT PV interval as compared to the PV interval promotes breaking of the retrograde conduction from the ventricles to the atria. This shorter duration of the PMT PV interval increases the chances that the retrograde conduction pathway from the ventricles to the atria is still in refractory when delivering the ventricular pacing pulse at the end of the PMT PV interval. Alternatively, even if the ventricular pacing pulse delivered at the end of the PMT PV interval is able to transfer through the retrograde conduction pathway to the atria, the subsequent retrograde atrial event is most likely falling into a post-ventricular atrial refractory period (PVARP) and is thereby not sensed by the aLP 102, or if sensed by the aLP 102, not communicated via i2i communication to the vLP 104.

Where the PV interval that the vLP 104 would normally use for ventricular pacing, if a PMT was not detected, is, e.g., 200 milliseconds (ms), then this means that a ventricular pacing pulse will be delivered 200 ms after an intrinsic atrial sensed event (aka a P-wave), if the PV interval expires without an intrinsic ventricular event being detected during the PV interval. An exemplary range for the PV interval is from 100 to 350 ms, which corresponds to atypical intrinsic atrial to ventricular conduction time. Lower and/or higher PV intervals are also possible, e.g., another exemplary range for the PV interval is from 340 to 500 ms,. The PMT PV interval, which is initiated in step S15, is shorter than the PV interval that the vLP 104 would otherwise use for ventricular pacing if a PMT was not detected. For example, the PMT PV interval can be 50 ms. An exemplary range for the PMT PV interval is from 25 ms to 150 ms, but is not limited thereto.

The PMT PV interval can be a programmed value, i.e., can be determined and programmed by a physician using an external device, such as programmer 106. Alternatively, the PMT PV interval could be defined constant duration, i.e., non-programmable. In other embodiments, the PMT PV interval is equal to PV interval minus a programmed or constant value, or the PMT PV interval can be a specified percentage of the of the PV interval.

Initiating the PMT PV interval in step S30 can involve the vLP 104 initiating a timer, which can be a dedicated PMT PV timer, or a same timer that is normally used for the PV interval can also be used for the PMT PV interval, depending upon the specific implementation.

The triggered PMT response can also involve the vLP 104 informing, in step S31, the aLP 102, via an i2i communication, of the detected PMT. For example, when the vLP 104 sends an i2i communication to the aLP 102, to inform the aLP 102 of a ventricular sensed event, the same i2i communication, or a following i2i communication, can inform the aLP 102 of the PMT detected by the vLP 104. However, given that the V2A i2i communication may still be compromised, the aLP 102 might not receive such an i2i communication.

Once the PMT PV interval is initiated at step S30 the vLP 104 monitors for any intrinsic ventricular event in step S32. If such an intrinsic ventricular event is detected in step S32 prior to the PMT PV interval expiring, which is indicative of the PMT having terminated, the vLP 104 terminates the PMT PV interval in step S36, notifies the aLP 102 of the ventricular sensed event via i2i communication and the flow then returns to step S22. Correspondingly, if the vLP 104 receives an i2i communication from the aLP 102 in step S33 indicating an intrinsic atrial event sensed by the aLP 102 prior to of the PMT PV interval expiring, which is indicative of the PMT having terminated, the vLP 104 will terminate the PMT PV interval in step S37 and the flow then returns to step S22. In these cases, the vLP 104 presumes that the PMT has been terminated. However, if the PMT PV interval expires without the detection of any intrinsic ventricular event by the vLP 104 or any intrinsic atrial event by the aLP 102 in step S34, the vLP 104 delivers, in step S35, a ventricular stimulation pulse to the ventricular cardiac chamber at the end of the PMT PV interval in an attempt to terminate the PMT. The vLP 104 also informs the aLP 102 of the ventricular paced event via an i2i communication. The flow then returns to step S22. If the PMT PV interval has not expired in step S34, the method returns to step S32.

The above-described operations thereby involve PMT monitoring and termination when the dual chamber leadless pacemaker system 100 is operating in the safeguard operational mode and when the i2i communication from the vLP 104 to the aLP 102 is compromised.

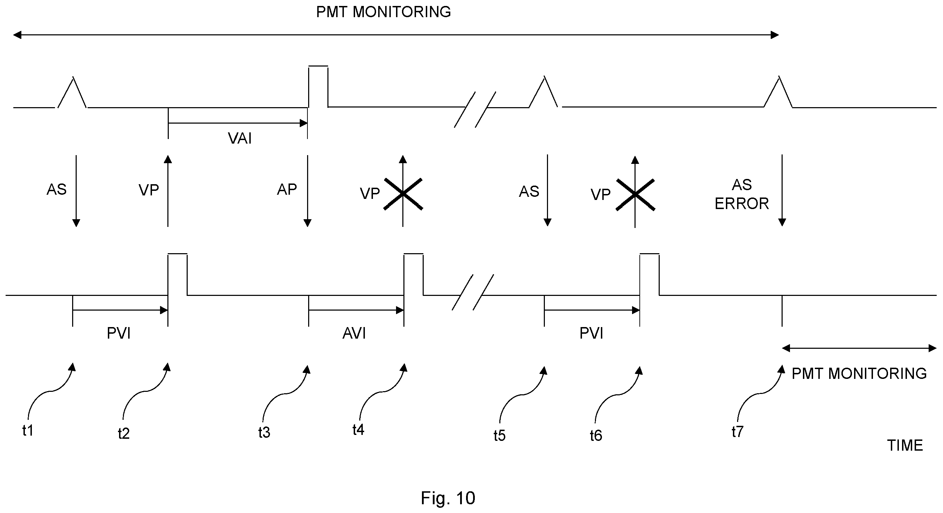

Fig. 10 is a timing diagram illustrating operations of an aLP 102 and a vLP 104 in connection with detection of compromised V2A i2i communication. The upper line is indicative of atrial events sensed or paced by the aLP 102 and the lower line is indicative of ventricular events sensed or paced by the vLP 104. At t1 in Fig. 8, the aLP 102 senses an atrial sensed (AS) event and sends an i2i communication to the vLP 104 to inform the vLP 104 of the AS event. The vLP 104, in response to being informed of the AS event, initiates a PV interval (PVI).

At time t2 the PVI expires and the vLP 104 delivers a ventricular pacing (VP) pulse and the vLP 104 sends an i2i communication to the aLP 102 to inform the aLP 102 of the VP event. The aLP 102, in response to being informed of the VP event, initiates a VA interval (VAI).

At time t3, the VAI expires and the aLP 102 delivers an atrial pacing (AP) pulse and sends an i2i communication to the vLP 104 to inform the vLP 104 of the AP event. The vLP 104, in response to being informed of the AP event, initiates an atrioventricular interval (AVI). This AVI may be of the same duration as the PVI or a different duration than the PVI.

At time t4 the AVI expires and the vLP 104 delivers a VP pulse and the vLP 104 sends an i2i communication to the aLP 102 to inform the aLP 102 of the VP event. However, at this point, the i2i communication sent by the vLP 104 is not successfully received by the aLP 104. The compromised V2A i2i communication continues for a number of cardiac cycles.

At time t5, the aLP 102 senses an atrial event and sends an i2i communication to the vLP 104 to inform the vLP 104 of the AS event. The vLP 104, in response to being informed of the AS event, initiates a PVI.

At time t6 the PVI expires and the vLP 104 delivers a VP pulse and the vLP 104 sends an i2i communication to the aLP 102 to inform the aLP 102 of the VP event. The aLP 102 still fails to receive the i2i communication from the vLP 104 and at this point the aLP 102 determines that the V2A i2i communication is compromised. Accordingly, the aLP 102 includes an error code or another form of indication of the compromised V2A i2i communication in a next i2i communication sent to the vLP 104 in response to sensing an atrial event at time t7. The aLP 102 further switches to the safeguard operational mode and discontinues the monitoring for PMT.

At time t7 and in response to the reception of the i2i communication with the error code or other form of indication of the compromised V2A i2i communication, the vLP 104 switches to the safeguard operational mode and assumes responsibility for monitoring for PMT.

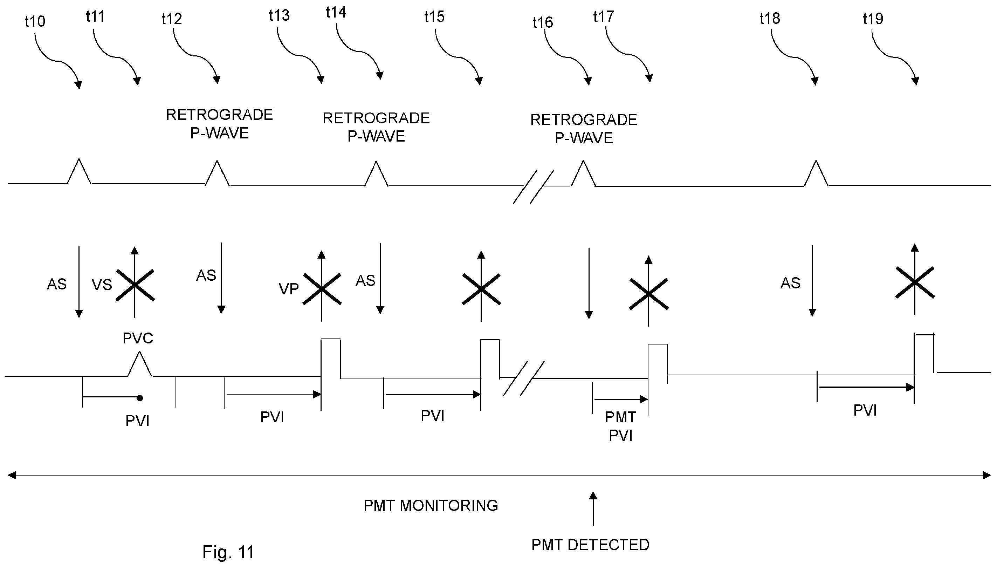

Fig. 11 is a timing diagram illustrating operations of an aLP 102 and a vLP 104 in connection with detection of a PMT by the vLP 104 while there is compromised V2A i2i communication.

At time t10, the aLP 102 senses an atrial event and sends an i2i communication to the vLP 104 to inform the vLP 104 of the AS event. The vLP 104, in response to being informed of the AS event, initiates a PVI.

At time t11, a premature ventricular contraction (PVC) occurs, which causes the vLP 104 to terminate the PVI and send an i2i communication to the aLP 102 to inform the aLP 102 of the ventricular sensed (VS) event, i.e., the PVC.

At time t12, a retrograde P-wave occurs due to retrograde conduction of the PVC from the ventricles to the atria. The retrograde P-wave is detected by the aLP 102 as an AS event, in response to which the aLP 102 sends an i2i communication to the vLP 104 to inform the vLP 104 of the AS event. The vLP 104, in response to being informed of the AS event, initiates a PVI.

At time t13, the PVI interval expires and the vLP 104 delivers a VP, and the vLP 104 sends an i2i communication to the aLP 102 to inform the aLP 102 of the VP event.

At time t14 another retrograde P-wave occurs. The retrograde P-wave is detected by the aLP 102 as an AS event, in response to which the aLP 102 sends an i2i communication to the vLP 104 to inform the vLP of the AS event. The vLP 104, in response to being informed of the AS event, initiates a PVI.

At time t15, the PVI interval expires and the vLP 104 delivers a VP, and the vLP 104 sends an i2i communication to the aLP 102 to inform the aLP 102 of the VP event. It is presumed that between times t15 and t16 additional retrograde P-waves occur (which are not shown), eventually resulting in the vLP 104 detecting a PMT, and triggering a PMT response, at time t16.

In response to the vLP 104 detecting the PMT at time t16, the vLP 104 initiates a PMT PVI that is shorter than the PVI that the vLP 104 uses for ventricular pacing when a PMT is not detected.

At time t17, the PMT PVI expires, in response to which the vLP 104 delivers a VP, and the vLP 104 informs the aLP 102, via an i2i communication, of the VP event. At this point it is presumed that the PMT has been terminated.

At time t18, the aLP 102 senses an atrial event and sends an i2i communication to the vLP 104 to inform the vLP 104 of the AS event. The vLP 104, in response to being informed of the AS event, initiates a PVI.

At time t19, the PVI expires, in response to which the vLP 104 delivers a VP and informs the aLP 102 of the VP event.

An aspect of the invention relates therefore to a dual chamber leadless pacemaker system 100, also referred to as a pacemaker system herein, see Fig. 1. The dual chamber leadless pacemaker system 100 comprises an aLP 102 configured to be implanted in or on an atrial cardiac chamber and comprising at least two electrodes 108, see Fig. 2, arranged to sense an intrinsic atrial event, pace the atrial cardiac chamber and perform i2i communication. The dual chamber leadless pacemaker system 100 also comprises a vLP 104 configured to be implanted in or on a ventricular cardiac chamber and comprising at least two electrodes 108, see Fig. 2, arranged to sense an intrinsic atrial event, pace the atrial cardiac chamber and perform i2i communication.

In an embodiment, the vLP 104 is configured to monitor for a PMT in response to reception of an i2i communication from the aLP 102 indicating compromised i2i communication from the vLP 104 to the aLP 102. The vLP 104 is configured to, following detection of a PMT, initiate a PMT PV interval in response to reception of an i2i communication from the aLP 102 indicating an atrial sensed event. The PMT PV interval is shorter than a PV interval that the vLP 104 would otherwise use for ventricular pacing if the PMT was not detected. The vLP 104 is, in this embodiment, also configured to deliver a ventricular pacing pulse to the ventricular cardiac chamber in response to the PMT PV interval expiring without an intrinsic ventricular event being detected during the PMT PV interval.

According to an embodiment, the vLP 104 assumes responsible for monitoring and detection of a PMT in response to reception of an i2i communication from the aLP 102 indicating that the V2A i2i communication from the vLP 104 to the aLP 102 is compromised or lost. Hence, the i2i communication indicates unsuccessful reception of i2i communications from the vLP 104 at the aLP 102, also referred to as loss of i2i communication from the vLP 104 at the aLP 102 herein.

The vLP 104, thus, initiates monitoring for any PMT once it has received the i2i communication from the aLP 102 indicating the compromised V2A i2i communication. If the vLP 104 detects such a PMT when it is responsible for PMT monitoring, the vLP 104 triggers a PMT response. The PMT response involves initiating the PMT PV interval in response to reception of an i2i communication from the aLP 102 indicating an AS event. In an embodiment, the PMT response also involves the vLP 104 transmitting an i2i communication to the aLP 104 indicating detection of PMT. This latter i2i communication is, though, most likely not received at the aLP 104 due to the compromised V2A i2i communications.

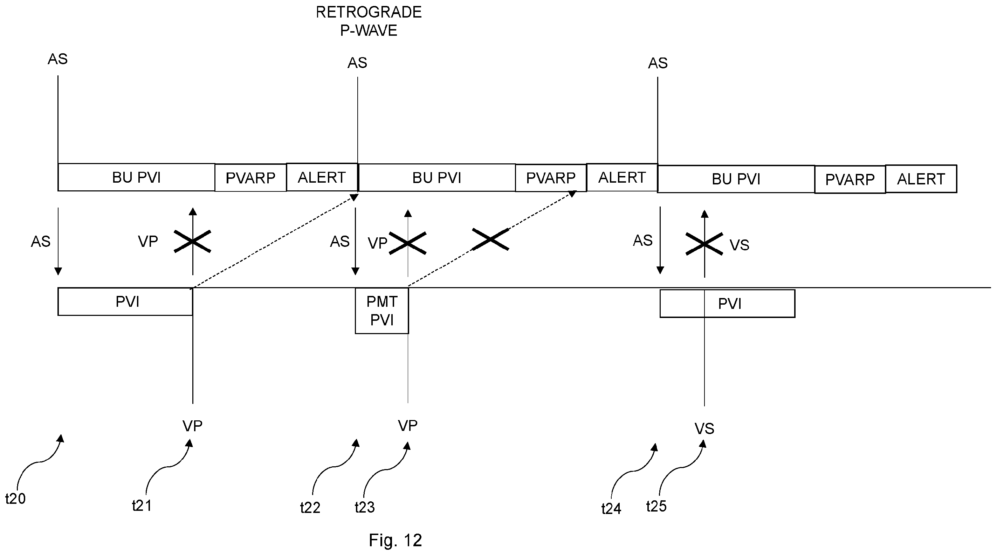

Fig. 12 is a timing diagram illustrating operations of an aLP 102 and a vLP 104 in connection with PMT detection by the vLP 104, during a period of time that the vLP 104 has taken over responsibility from the aLP 102 for PMT monitoring because the aLP 102 had detected compromised V2A i2i communication.

At time t20, the aLP 102 senses an atrial event and initiates a back-up (BU) PVI and sends an i2i communication to the vLP 104 to inform the vLP 104 of the AS event. The vLP 104, in response to being informed of the AS event, initiates a PVI.

At time t21, the PVI interval expires and the vLP 104 delivers a VP, and the vLP 104 sends an i2i communication to the aLP 102 to inform the aLP 102 of the VP event. However, as indicated by the X, the i2i communication from the vLP 104 to the aLP 102 at time t21 is not received by the aLP 102 because of the compromised V2A i2i communications.

At time t22 a retrograde P-wave occurs caused by retrograde conduction of the VP delivered at time t21, which is indicated by the hatched arrow in Fig. 10. The retrograde P-wave is detected by the aLP 102 as an AS event, in response to which the aLP 102 sends an i2i communication to the vLP 104 to inform the vLP of the AS event. The aLP 102 also initiates a BU PVI. At this point, the vLP 104 detects a PMT. Accordingly, the vLP 104, in response to being informed of the AS event, initiates a PMT PVI rather than a PVI.

At time t23, the PMT PVI expires, in response to which the vLP 104 delivers a VP, and the vLP 104 informs the aLP 102, via an i2i communication, of the VP event. However, as indicated by the X, the i2i communication from the vLP 104 to the aLP 102 at time t23 is not received by the aLP 102 because of the compromised V2A i2i communications. The duration of the PMT PVI is shorter than the duration of the PVI. In the illustrative example of Fig. 10, the shortened PVI effectively breaks the retrograde conduction. At this point, the PMT is presumed to be terminated.

As a consequence, a next intrinsic atrial event (i.e., the next AS event), which is a true P-wave and not any retrograde P-wave, is detected by the aLP 102 in a so-called atrial alert period following expiry of a postventricular atrial refractory period (PVARP) following expiry of the BU PVI as indicated in Fig. 12.

Atrial alert period as referred to herein refers to a time period when the aLP 102 listens for, i.e., senses, and can respond to atrial signals.

The aLP 102 initiates the BU PVI in response to sensing the AS event and sends an i2i communication to the vLP 104 to inform the vLP 104 of the AS event. The vLP 104, in response to being informed of the AS event, initiates a PVI.

At time t25, the vLP 104 senses a ventricular event, terminates the PVI and sends an i2i communication to the aLP 102 to inform the aLP 102 of the VS event. However, as indicated by the X, the i2i communication from the vLP 104 to the aLP 102 at time t25 is not received by the aLP 102 because of the compromised V2A i2i communications.

In this embodiment, the PMT PV interval has a duration selected so that an end of the PMT PV interval occurs while a retrograde conduction pathway from the ventricular cardiac chamber to the atrial cardiac chamber is in refractory.

In an alternative embodiment, the shortened PVI, i.e., PMT PVI, effectively breaks the retrograde conduction as discussed above. However, in this embodiment, the aLP 102 delivers an atrial pacing pulse so that a next intrinsic P-wave occurs during a refractory period. Once the vLP 104 detects this, the vLP 104 will inhibit delivery of a ventricular pacing pulse for one cardiac cycle. This will allow the atrial cardiac chamber to repolarize and for an intrinsic P-wave to occur during a next alert period. Once that occur, the vLP 104 will continue ventricular sensing and pacing operation in the normal operational mode.

As mentioned in the foregoing, the aLP 102 preferably withholds atrial pacing when operating in the safeguard operational mode. This means that the aLP 102 generally does not deliver any atrial pacing or stimulation pulse to the atrium when operating in the safeguard operational mode. In the embodiment discussed above, the aLP 102, however, delivers an atrial pacing pulse so that a next intrinsic P-wave occurs during an atrial refractory period. Such an atrial pacing pulse delivered by the aLP 102 when operating in the safeguard operational mode is also referred to as a withheld atrial pacing pulse herein to indicate that the aLP 102 typically does not deliver any atrial pacing pulses, i.e., atrial pacing is withheld, during the safeguard operational mode.

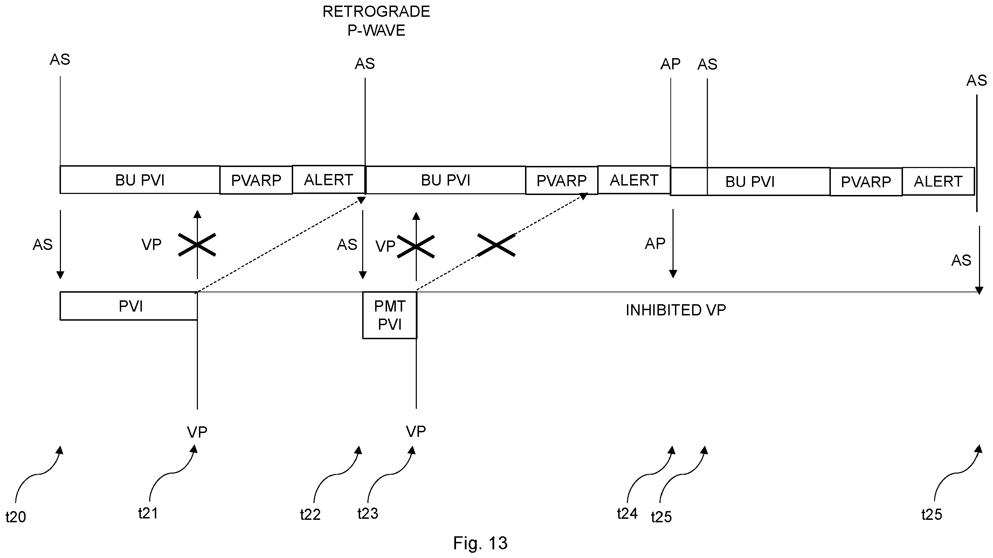

Such an embodiment is shown in Fig. 13. The operation in this embodiment is similar to Fig. 12 up to time t24.

At time t24, the aLP 102 delivers a (withheld) atrial pacing pulse at the end of the atrial alert period and sends an i2i communication to the vLP 104 to inform the vLP 104 of the (withheld) AP event. The vLP 104, in response to being informed of the withheld AP event, inhibits ventricular pacing for one cardiac cycle.

At time t25, a next P-wave occurs during an atrial refractory period following delivery of the (withheld) AP, wherein the atrial refractory period refers to a period during which the aLP 102 disables sensing for an intrinsic atrial event. This means that this next P-wave is not sensed by the aLP 102 or, if sensed, the aLP 102 does not inform the vLP 104 of the sensed P-wave.

At time t26, the aLP 102 senses an atrial event and initiates the BU PVI and sends an i2i communication to the vLP 104 to inform the vLP 104 of the AS event. The vLP 104, in response to being informed of the AS event, initiates a PVI.

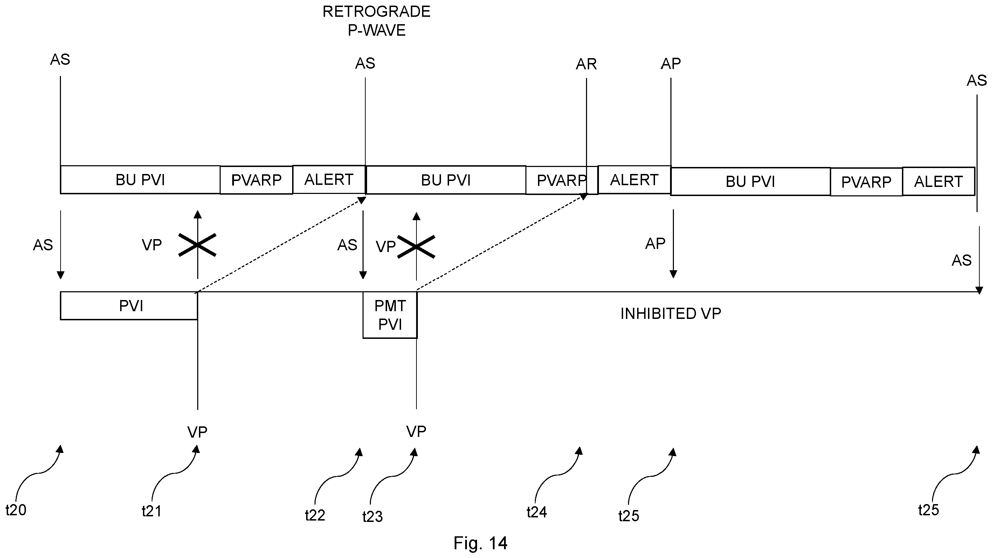

Fig. 14 illustrates another embodiment. In this embodiment, the shortened PVI, i.e., PMT PVI, causes a retrograde P-wave (denoted AR in Fig. 14) to occur during the atrial refractory period. In this embodiment, the next atrial event may be the delivery of a withheld atrial pacing pulse similar to Fig. 13, which causes the vLP 104 to inhibit ventricular pacing for one cardiac cycle. This will allow the atrial cardiac chamber to repolarize and for an intrinsic P-wave to occur during a next alert period. Once that occur, the vLP 104 will continue ventricular sensing and pacing operation.

The operation in this embodiment is similar to Figs. 12 and 13 up to time t24.

At time t24, the retrograde P-wave induced by the ventricular pacing pulse delivered by the vLP 104 at the end of the PMT PVI at time t23 occurs. However, this retrograde P-wave occurs during the PVARP following expiry of the BU PVI as indicated in Fig. 14. In an embodiment, during this PVARP, the aLP 102 does not sense for any intrinsic atrial activity and the retrograde P-wave will therefore not be sensed or detected by the aLP 102. In another embodiment, during this PVARP, the aLP 102 senses for intrinsic atrial activity but, in response to sensing such an intrinsic atrial activity, does not send any i2i communication to the vLP 104 indicating the AS event. This means that the retrograde P-wave occurring during the PVARP will not cause the vLP 104 to initiate any PVI (or PMT PVI).

At time t25, the aLP 102 delivers a withheld atrial pacing pulse at the end of the atrial alert period and sends an i2i communication to the vLP 104 to inform the vLP 104 of the withheld AP event. The vLP 104, in response to being informed of the withheld AP event, inhibits ventricular pacing for one cardiac cycle.

At time t26, the aLP 102 senses an atrial event and initiates the BU PVI and sends an i2i communication to the vLP 104 to inform the vLP 104 of the AS event. The vLP 104, in response to being informed of the AS event, initiates a PVI.

Hence, in an embodiment, the aLP 102 is configured to initiate an atrial refractory period in response to sensing an intrinsic atrial event and the aLP 102 is configured to not transmit any i2i communication indicating an atrial sensed event to the vLP 104 if the aLP 102 senses a further intrinsic atrial event during the atrial refractory period. In this embodiment, the PMT PV interval has a duration selected so that any retrograde P wave, induced by the ventricular pacing pulse delivered by the vLP 104 to the ventricular cardiac chamber in response to the PMT PV interval expiring without an intrinsic ventricular event being detected during the PMT PV interval, falls within the atrial refractory period.

In an embodiment, the aLP 102 is configured to initiate an atrial refractory period in response to sensing an intrinsic atrial event. The aLP is, in this embodiment, also configured to not transmit any i2i communication indicating an atrial sensed event to the vLP 104 if the aLP 102 senses a further intrinsic atrial event during the atrial refractory period. The aLP is further configured to initiate an atrial alert period in response to the atrial refractory period expiring and deliver a (withheld) atrial pacing pulse to the atrial cardiac chamber in response to the atrial alert period expiring.

In an embodiment, the aLP 102 is configured to transmit an i2i communication to the vLP 104 indicating the delivery of the (withheld) atrial pacing pulse to the atrial cardiac chamber. The vLP 104 is configured to, in response to reception of the i2i communication from the aLP 102 indicating the delivery of the (withheld) atrial pacing pulse to the atrial cardiac chamber, inhibit delivery of a ventricular pacing pulse to the ventricular cardiac chamber for a cardiac cycle.

In an embodiment, the vLP 104 is configured to, following delivery of the ventricular pacing pulse to the ventricular cardiac chamber in response to the PMT PV interval expiring without an intrinsic ventricular event being from the aLP 102 indicating an atrial sensed event or an atrial paced event.

Thus, once a ventricular pacing pulse has been delivered by the vLP 104 at the end of the PMT PV interval, the vLP 104 presumes that the PMT has been terminated and thereby continues by initiating the PVI interval rather than the PMT PV interval in response to reception of an i2i communication from the aLP 102 indicating an AS event or an AP event.

In the case normal, i.e., non-PMT, operation resumes in the safeguard operational mode, and the vLP 104 re-detects PMT, the vLP 104 could keep the shortened PVI, i.e., PMT PVI, in effect for a period of time and then gradually increase the PMT PVI while monitoring for PMT. If the interval is lengthened and a PMT is detected, the vLP 104 can terminate the PMT as disclosed herein and then go back to using the PV interval that it was using previously to prevent PMT from re-occurring.

Hence, in an embodiment, the vLP 104 is configured to, in response to detection of a subsequent PMT, initiate the PMT PV interval in response to reception of an i2i communication from the aLP 102 indicating an atrial sensed event. The vLP 104 is also configured to, following delivery of the ventricular pacing pulse to the ventricular cardiac chamber in response to the PMT PV interval expiring without an intrinsic ventricular event being detected during the PMT PV interval, gradually increase a duration of the PMT PV interval while monitoring for another subsequent PMT. The vLP 104 is, in this embodiment, configured to, in response to detection of the another subsequent PMT, set a duration of a PV interval that the vLP 104 otherwise uses for ventricular pacing if the PMT was not detected to be equal to or longer than the PMT PV interval but shorter than the increased PMT PV interval used when the another subsequent PMT is detected.

This embodiment thereby sets the PV interval to be used by the vLP 104 during normal, i.e., non-PMT, operation to have a duration that is longer than the PMT PV interval but shorter than the original PV interval used by the vLP 104. Such a shorter PV interval reduces the risk of PMT re-occurring as compared to going back to the longer PV interval.

In an embodiment, the aLP 102 is configured to monitor for PMT when i2i communication from the vLP 104 to the aLP 102 is functional. The aLP 102 is also configured to, in response to detection of compromised i2i communication from the vLP 104 to the aLP 102, transmit the i2i communication to the vLP 104 indicating compromised i2i communication from the vLP 104 to the aLP 102. In this embodiment, the aLP 102 is configured to interrupt its monitoring for PMT until i2i communication from the vLP 104 to the aLP 102 is functional.

Hence, in this embodiment, the aLP 102 is responsible for PMT monitoring during normal operational mode when the V2A i2i communication is functional, i.e., i2i communications sent by the vLP 104 are correctly received at the aLP 102. However, once the aLP 102 detects compromised V2A i2i communication it informs the vLP 104 accordingly and the responsibility for PMT monitoring is switched from the aLP 102 to the vLP 104.

In an embodiment, the aLP 102 is configured to switch from collectively operating with the vLP 104 in a DDD or a DDD(R) pacing mode to a VDD or a VDD(R) pacing mode in response to detection of compromised i2i communication from the vLP 104 to the aLP 102 and the vLP 104 is configured to switch from collectively operating with the aLP 102 in the DDD or the DDD(R) pacing mode to the VDD or the VDD(R) pacing mode in response to reception of the i2i communication from the aLP 102 indicating compromised i2i communication from the vLP 104 to the aLP 102.

In this embodiment, the normal operational mode for the dual chamber leadless pacemaker system 100 is, thus, a DDD or a DDD(R) pacing mode, whereas the safeguard operational mode is a VDD or the VDD(R) pacing mode.

PMT can be detected by the vLP 104 by detection of VP-AS intervals for a number of consecutive cardiac cycles and when the ventricular pacing rate for each of these consecutive cardiac cycles exceeds a PMT detection rate.

Thus, in an embodiment, the vLP 104 is configured to monitor for VP-AS intervals for a predefined number of consecutive cardiac cycles when a pacing rate exceeds a PMT detection rate. Each of the VP-AS intervals represents a duration from delivery of a ventricular pacing pulse to the ventricular cardiac chamber by the vLP 104 to reception of an i2i communication from the aLP 102 indicating an atrial sensed event. The vLP is configured to detect a PMT based on the monitored VP-AS intervals when the pacing rate exceeds the PMT detection rate.

In an embodiment, see Fig. 2, the aLP 102 further comprises at least one receiver 120, 122 connected to the at least two electrodes 108 and configured to receive i2i communications from the vLP 104. The aLP 102 also comprises sensing circuitry 132 connected to the at least two electrodes 108 and configured to sense intrinsic atrial events and at least one pulse generator 116 connected to the at least two electrodes 108 and configured to generate pacing pulses and i2i communications pulses. The aLP 102 further comprises a controller 112 connected to the at least one receiver 120, 122, the sensing circuitry 132 and the at least one pulse generator 116 and configured to control the at least one receiver 120, 122, the sensing circuitry 132 and the at least one pulse generator 116. In this embodiment, the vLP 104 further comprises at least one receiver 120, 122 connected to the at least two electrodes 108 and configured to receive i2i communications from the aLP 102. The vLP 104 also comprises sensing circuitry 132 connected to the at least two electrodes 108 and configured to sense intrinsic ventricular events and at least one pulse generator 116 connected to the at least two electrodes 108 and configured to generate pacing pulses and i2i communications pulses. The vLP 104 further comprises a controller 112 connected to the at least one receiver 120, 122, the sensing circuitry 132 and the pulse generator 116 and configured to control the at least one receiver 120, 122, the sensing circuitry 132 and the pulse generator 116.

In an embodiment, the aLP 102 and the vLP 104 each comprises a respective pulse generator 116 configured to generate both the pacing pulses and the i2i communication pulses. In another embodiment, the aLP 102 and/or the vLP 104 comprises a first pulse generator configured to generate the pacing pulses and a second pulse generator configured to generate the i2i communication pulses.

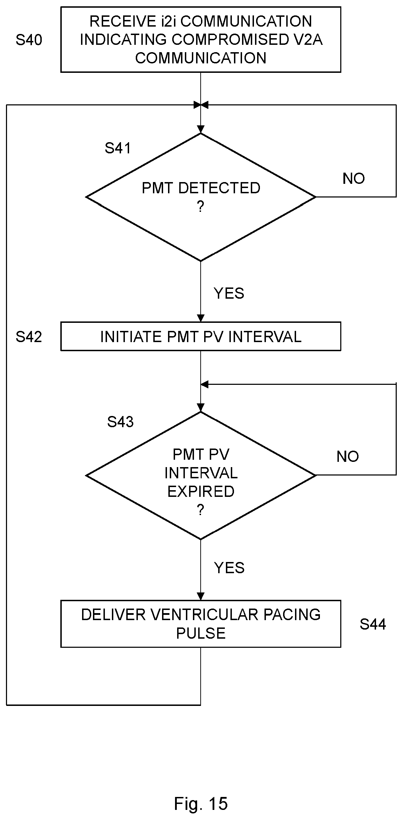

A method is provided for terminating a PMT by a dual chamber leadless pacemaker system 100 comprising an aLP 102 configured to be implanted in or on an atrial cardiac chamber and comprising at least two electrodes 108 arranged to sense an intrinsic atrial event, pace the atrial cardiac chamber and perform i2i communication and a vLP 104 configured to be implanted in or on a ventricular cardiac chamber and comprising at least two electrodes 108 arranged to sense an intrinsic ventricular event, pace the ventricular cardiac chamber and perform i2i communication. The method comprises, see Fig. 15, the vLP 104 monitoring, in step S41, for a PMT in response to reception, in step S40, of an i2i communication from the aLP 102 indicating compromised i2i communication from the vLP 104 to the aLP 102. The method also comprises the vLP 104 initiating, in step S42 and following detection of a PMT in step S41, a PMT PV interval in response to reception of an i2i communication from the aLP 102 indicating an atrial sensed event. The PMT PV interval is shorter than a PV interval that the vLP 104 would otherwise use for ventricular pacing if the PMT was not detected. The method further comprises the vLP 104 delivering, in step S44, a ventricular pacing pulse to the ventricular cardiac chamber in response to the PMT PV interval expiring without an intrinsic ventricular event being detected during the PMT PV interval as determined in step S43.

In an embodiment, the method further comprises the aLP 102 initiating an atrial refractory period in response to sensing an intrinsic atrial event. In this embodiment, the aLP 102 refrains from transmitting any i2i communication indicating an atrial sensed event to the vLP 104 if the aLP 102 senses a further intrinsic atrial events during the atrial refractory period. The aLP 102 initiates an atrial alert period in response to the atrial refractory period expiring and the aLP 102 delivers a (withheld) atrial pacing pulse to the atrial cardiac chamber in response to the atrial alert period expiring.

In a particular embodiment, the aLP 102 transmits an i2i communication to the vLP 104 indicating the delivery of the (withheld) atrial pacing pulse to the atrial cardiac chamber. In this embodiment, the vLP 104 inhibits, in response to reception of the i2i communication from the aLP 102 indicating the delivery of the (withheld) atrial pacing pulse to the atrial cardiac chamber, delivery of a ventricular pacing pulse to the ventricular cardiac chamber for a cardiac cycle.

In an embodiment, the method further comprises the vLP 104 initiating, following delivery of the ventricular pacing pulse to the ventricular cardiac chamber in response to the PMT PV interval expiring without an intrinsic ventricular event being detected during the PMT PV interval, the PV interval in response to reception of an i2i communication from the aLP 102 indicating an atrial sensed event or an atrial paced event.

In an embodiment, the method further comprises the vLP 104 initiating, in response to detection of a subsequent PMT, the PMT PV interval in response to reception of an i2i communication from the aLP 102 indicating an atrial sensed event. In this embodiment, the vLP 104 gradually increases, following delivery of the ventricular pacing pulse to the ventricular cardiac chamber in response to the PMT PV interval expiring without an intrinsic ventricular event being detected during the PMT PV interval, a duration of the PMT PV interval while monitoring for another subsequent PMT. The vLP 104 also sets, in response to detection of the another subsequent PMT, a duration of a PV interval that the vLP 104 otherwise uses for ventricular pacing if the PMT was not detected to be equal to or longer than the PMT PV interval but shorter than the increased PMT PV interval used when the another subsequent PMT is detected.

In an embodiment, the method further comprises the aLP 102 monitoring for PMT when i2i communication from the vLP 104 to the aLP 102 is functional. In this embodiment, the aLP 102 transmits, in response to detection of compromised i2i communication from the vLP 104 to the aLP 102, the i2i communication to the vLP 104 indicating compromised i2i communication from the vLP 102 to the aLP 102. The aLP 102 further interrupts its monitoring for PMT until i2i communication from the vLP 104 to the aLP 102 is functional.

In an embodiment, the method further comprises the aLP 102 switching from collectively operating with the vLP 104 in a DDD or a DDD(R) pacing mode to a VDD or a VDD(R) pacing mode in response to detection of compromised i2i communication from the vLP 104 to the aLP 102, and the vLP 104 switching from collectively operating with the aLP 102 in the DDD or the DDD(R) pacing mode to the VDD or the VDD(R) pacing mode in response to reception of the i2i communication from the aLP 102 indicating compromised i2i communication from the vLP 104 to the aLP 102.

In an embodiment, the method further comprises the aLP 102 initiating an atrial refractory period in response to sensing an intrinsic atrial event. The aLP 102 refrains from transmitting any i2i communication indicating an AS event to the vLP 104 if the aLP 102 senses a further intrinsic atrial event during the atrial refractory period. In this embodiment, the PMT PV interval has a duration selected so that any retrograde P wave, induced by the ventricular pacing pulse delivered by the vLP 104 to the ventricular cardiac chamber in response to the PMT PV interval expiring without an intrinsic ventricular event being detected during the PMT PV interval, falls within the atrial refractory period.

In an embodiment, the PMT PV interval has a duration selected so that an end of the PMT PV interval occurs while a retrograde conduction pathway from the ventricular cardiac chamber to the atrial cardiac chamber is in refractory.

In an embodiment, the method further comprises the vLP 104 monitoring for VP-AS intervals for a predefined number of consecutive cardiac cycles when a pacing rate exceeds a PMT detection rate, and the vLP 104 detecting a PMT based on the monitored VP-AS intervals when the pacing rate exceeds the PMT detection rate.

A further aspect of the invention relates to a dual chamber leadless pacemaker system 100 comprising an aLP 102 configured to be implanted in or on an atrial cardiac chamber and comprising at least two electrodes 108 arranged to sense an intrinsic atrial event, pace the atrial cardiac chamber and perform i2i communication, and a VLP 104 configured to be implanted in or on a ventricular cardiac chamber and comprising at least two electrodes 108 arranged to sense an intrinsic ventricular event, pace the ventricular cardiac chamber and perform i2i communication. The aLP 102 is configured to monitor for a PMT based on intrinsic atrial events detected by the aLP 102 and i2i communications from the vLP 104 indicating VP events. The aLP 102 is configured to, in response to detection of compromised i2i communication from the vLP 104 to the aLP 102, transmit an i2i communication to the vLP 104 indicating compromised i2i communication from the vLP 104 to the aLP 102. The aLP 102 is also configured to interrupt its monitoring for PMT until i2i communication from the vLP 104 to the aLP 102 is functional. The vLP 104 is configured to, in response to reception of the i2i communication from the aLP 102 indicating compromised i2i communication from the vLP 104 to the aLP 102, monitor for a PMT based on VP events caused by the vLP 104 and i2i communications from the aLP 102 indicating events.

In an embodiment, the aLP 102 is configured to monitor for VP-AS intervals for a predefined number of consecutive cardiac cycles when a pacing rate exceeds a PMT detection rate. Each of the VP-AS intervals represents, from the aLP 102 perspective, a duration from reception of an i2i communication from the vLP 104 indicating a ventricular paced event to sensing an intrinsic atrial event by the aLP 102. The aLP 102 is also configured to detect a PMT based on the monitored VP-AS intervals when the pacing rate exceeds the PMT detection rate.

In an embodiment, the vLP 104 is configured to monitor for VP-AS intervals for a predefined number of consecutive cardiac cycles when a pacing rate exceeds a PMT detection rate. Each of the VP-AS intervals represents, from the vLP 104 perspective, a duration from delivery of a ventricular pacing pulse to the ventricular cardiac chamber by the vLP 104 to reception of an i2i communication from the aLP 102 indicating an atrial sensed event. The vLP 104 is also configured to detect a PMT based on the monitored VP-AS intervals when the pacing rate exceeds the PMT detection rate.

A method is provided for terminating a PMT by a dual chamber leadless pacemaker system 100 comprising an aLP 102 configured to be implanted in or on an atrial cardiac chamber and comprising at least two electrodes 108 arranged to sense an intrinsic atrial event, pace the atrial cardiac chamber and perform i2i communication, and a vLP 104 configured to be implanted in or on a ventricular cardiac chamber and comprising at least two electrodes 108 arranged to sense an intrinsic ventricular event, pace the ventricular cardiac chamber and perform i2i communication. The method comprises the aLP 102 monitoring for a PMT based on intrinsic atrial events detected by the aLP 102 and i2i communications from the vLP 104 indicating VP events. The method also comprises the aLP 102 transmitting, in response to detection of compromised i2i communication from the vLP 104 to the aLP 102, an i2i communication to the vLP 104 indicating compromised i2i communication from the vLP 104 to the aLP 102. The method further comprises the aLP 102 interrupting its monitoring for PMT until i2i communication from the vLP 104 to the aLP 102 is functional. The method additionally comprises the vLP 104 monitoring, in response to reception of the i2i communication from the aLP 102 indicating compromised i2i communication from the vLP 104 to the aLP 102, for a PMT based on ventricular paced events caused by the vLP 104 and i2i communications from the aLP 102 indicating atrial sensed events.

In an embodiment, the method further comprises the aLP 102 monitoring for VP-AS intervals for a predefined number of consecutive cardiac cycles when a pacing rate exceeds a PMT detection rate. Each of the VP-AS intervals represents, from the aLP 102 perspective, a duration from reception of an i2i communication from the vLP 104 indicating a ventricular paced event to sensing an intrinsic atrial event by the aLP 102. The method also comprises the aLP 102 detecting a PMT based on the monitored VP-AS intervals when the pacing rate exceeds the PMT detection rate.

In an embodiment, the method comprises the vLP 104 monitoring for VP-AS intervals for a predefined number of consecutive cardiac cycles when a pacing rate exceeds a PMT detection rate. Each of the VP-AS intervals represents, from the vLP 104 perspective, a duration from delivery of a ventricular pacing pulse to the ventricular cardiac chamber by the vLP 104 to reception of an i2i communication from the aLP indicating an atrial sensed event. The method also comprises the vLP 104 detecting a PMT based on the monitored VP-AS intervals when the pacing rate exceeds the PMT detection rate.

The various embodiments discussed in the foregoing in connection with Figs. 1 to 12 also apply to these aspects of the invention.

It is to be understood that the subject matter described herein is not limited in its application to the details of construction and the arrangement of components set forth in the description herein or illustrated in the drawings hereof. The subject matter described herein is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Further, it is noted that the term "based on" as used herein, unless stated otherwise, should be interpreted as meaning based at least in part on, meaning there can be one or more additional factors upon which a decision or the like is made. For example, if a decision is based on the results of a comparison, that decision can also be based on one or more other factors in addition to being based on results of the comparison.

It is to be understood that the above description is intended to be illustrative, and not restrictive. For example, the above-described embodiments (and/or aspects thereof) may be used in combination with each other. In addition, many modifications may be made to adapt a particular situation or material to the teachings of the embodiments of the present technology without departing from its scope. While the dimensions, types of materials and coatings described herein are intended to define the parameters of the embodiments of the present technology, they are by no means limiting and are exemplary embodiments. Many other embodiments will be apparent to those of skill in the art upon reviewing the above description. The scope of the embodiments of the present technology should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, the terms "first," "second," and "third," etc. are used merely as labels, and are not intended to impose numerical requirements on their objects. Further, the limitations of the following claims are not written in means-plus-function format and are not intended to be interpreted based on 35 U.S.C. § 112(f), unless and until such claim limitations expressly use the phrase "means for" followed by a statement of function void of further structure.

## Claims

1. A dual chamber leadless pacemaker system (100) comprising:
an atrial leadless pacemaker, aLP, (102) configured to be implanted in or on an atrial cardiac chamber and comprising at least two electrodes (108) arranged to sense an intrinsic atrial event, pace the atrial cardiac chamber and perform implant-to-implant (i2i) communication; and
a ventricular leadless pacemaker, vLP, (104) configured to be implanted in or on a ventricular cardiac chamber and comprising at least two electrodes (108) arranged to sense an intrinsic ventricular event, pace the ventricular cardiac chamber and perform i2i communication, **characterized in that**
the vLP (104) is configured to monitor for a pacemaker mediated tachycardia (PMT) in response to reception of an i2i communication from the aLP (102) indicating compromised i2i communication from the vLP (104) to the aLP (102);
the vLP (104) is configured to, following detection of a PMT, initiate a PMT PV interval in response to reception of an i2i communication from the aLP (102) indicating an atrial sensed event, wherein the PMT PV interval is shorter than a PV interval that the vLP (104) would otherwise use for ventricular pacing if the PMT was not detected; and
the vLP (104) is configured to deliver a ventricular pacing pulse to the ventricular cardiac chamber in response to the PMT PV interval expiring without an intrinsic ventricular event being detected during the PMT PV interval.

2. The dual chamber leadless pacemaker system (100) according to claim 1, wherein:
the aLP (102) is configured to initiate an atrial refractory period in response to sensing an intrinsic atrial event;
the aLP (102) is configured to not transmit any i2i communication indicating an atrial sensed event to the vLP (104) if the aLP (102) senses a further intrinsic atrial event during the atrial refractory period;
the aLP (102) is configured to initiate an atrial alert period in response to the atrial refractory period expiring; and
the aLP (102) is configured to deliver an atrial pacing pulse to the atrial cardiac chamber in response to the atrial alert period expiring.

3. The dual chamber leadless pacemaker system (100) according to claim 2, wherein:
the aLP (102) is configured to transmit an i2i communication to the vLP (104) indicating the delivery of the atrial pacing pulse to the atrial cardiac chamber; and
the vLP (104) is configured to, in response to reception of the i2i communication from the aLP (102) indicating the delivery of the atrial pacing pulse to the atrial cardiac chamber, inhibit delivery of a ventricular pacing pulse to the ventricular cardiac chamber for a cardiac cycle.

4. The dual chamber leadless pacemaker system (100) according to any one of claims 1 through 3, wherein the vLP (104) is configured to, following delivery of the ventricular pacing pulse to the ventricular cardiac chamber in response to the PMT PV interval expiring without an intrinsic ventricular event being detected during the PMT PV interval, initiate the PV interval in response to reception of an i2i communication from the aLP (102) indicating an atrial sensed event or an atrial paced event.

5. The dual chamber leadless pacemaker system (100) according to any one of claims 1 through 4, wherein:
the vLP (104) is configured to, in response to detection of a subsequent PMT, initiate the PMT PV interval in response to reception of an i2i communication from the aLP (102) indicating an atrial sensed event;
the vLP (104) is configured to, following delivery of the ventricular pacing pulse to the ventricular cardiac chamber in response to the PMT PV interval expiring without an intrinsic ventricular event being detected during the PMT PV interval, gradually increase a duration of the PMT PV interval while monitoring for another subsequent PMT; and
the vLP (104) is configured to, in response to detection of the another subsequent PMT, set a duration of a PV interval that the vLP (104) otherwise uses for ventricular pacing if the PMT was not detected to be equal to or longer than the PMT PV interval but shorter than the increased PMT PV interval used when the another subsequent PMT is detected.

6. The dual chamber leadless pacemaker system (100) according to any one of claims 1 through 5, wherein:
the aLP (102) is configured to monitor for a PMT when i2i communication from the vLP (104) to the aLP (102) is functional;
the aLP (102) is configured to, in response to detection of compromised i2i communication from the vLP (104) to the aLP (102), transmit an i2i communication to the vLP (104) indicating compromised i2i communication from the vLP (104) to the aLP (102); and
the aLP (102) is configured to interrupt its monitoring for a PMT until i2i communication from the vLP (104) to the aLP (102) is functional.

7. The dual chamber leadless pacemaker system (100) according to any one of claims 1 through 6, wherein:
the aLP (102) is configured to switch from collectively operating with the vLP (104) in a DDD or a DDD(R) pacing mode to a VDD or a VDD(R) pacing mode in response to detection of compromised i2i communication from the vLP (104) to the aLP (102); and
the vLP (104) is configured to switch from collectively operating with the aLP (102) in the DDD or the DDD(R) pacing mode to the VDD or the VDD(R) pacing mode in response to reception of an i2i communication from the aLP (102) indicating compromised i2i communication from the vLP (104) to the aLP (102).

8. The dual chamber leadless pacemaker system (100) according to any one of claims 1 through 7, wherein:
the aLP (102) is configured to initiate an atrial refractory period in response to sensing an intrinsic atrial event;
the aLP (102) is configured to not transmit any i2i communication indicating an atrial sensed event to the vLP (104) if the aLP (102) senses a further intrinsic atrial event during the atrial refractory period; and
the PMT PV interval has a duration selected so that any retrograde P wave, induced by the ventricular pacing pulse delivered by the vLP (104) to the ventricular cardiac chamber in response to the PMT PV interval expiring without an intrinsic ventricular event being detected during the PMT PV interval, falls within the atrial refractory period.

9. The dual chamber leadless pacemaker system (100) according to any one of claims 1 through 8, wherein the the PMT PV interval has a duration selected so that an end of the PMT PV interval occurs while a retrograde conduction pathway from the ventricular cardiac chamber to the atrial cardiac chamber is in refractory.

10. The dual chamber leadless pacemaker system (100) according to any one of claims 1 through 9, wherein:
the vLP (104) is configured to monitor for VP-AS intervals for a predefined number of consecutive cardiac cycles when a pacing rate exceeds a PMT detection rate, wherein each of the VP-AS intervals represents a duration from delivery of a ventricular pacing pulse to the ventricular cardiac chamber by the vLP (104) to reception of an i2i communication from the aLP (102) indicating an atrial sensed event; and
the vLP (104) is configured to detect a PMT based on the monitored VP-AS intervals when the pacing rate exceeds the PMT detection rate.

11. The dual chamber leadless pacemaker system (100) according to any one of claims 1 through 10, wherein:
the aLP (102) further comprises:
at least one receiver (120, 122) connected to the at least two electrodes (108) and configured to receive i2i communications from the vLP (104);
a sensing circuitry (132) connected to the at least two electrodes (108) and configured to sense intrinsic atrial events;
at least one pulse generator (116) connected to the at least two electrodes (108) and configured to generate pacing pulses and i2i communication pulses; and
a controller (112) connected to the at least one receiver, the sensing circuitry and the at least one pulse generator and configured to control the at least one receiver, the sensing circuitry and the at least one pulse generator; and
the vLP (104) further comprises:
at least one receiver (120, 122) connected to the at least two electrodes (108) and configured to receive i2i communications from the aLP (102);
a sensing circuitry (132) connected to the at least two electrodes (108) and configured to sense intrinsic ventricular events;
at least one pulse generator (116) connected to the at least two electrodes (108) and configured to generate pacing pulses and i2i communication pulses; and
a controller (112) connected to the at least one receiver (120, 122), the sensing circuitry (132) and the at least one pulse generator (116) and configured to control the at least one receiver (120, 122), the sensing circuitry (132) and the at least one pulse generator (116).

12. A dual chamber leadless pacemaker system (100) comprising:
an atrial leadless pacemaker, aLP (102), configured to be implanted in or on an atrial cardiac chamber and comprising at least two electrodes (108) arranged to sense an intrinsic atrial event, pace the atrial cardiac chamber and perform implant-to-implant (i2i) communication; and
a ventricular leadless pacemaker, vLP, (104) configured to be implanted in or on a ventricular cardiac chamber and comprising at least two electrodes (108) arranged to sense an intrinsic ventricular event, pace the ventricular cardiac chamber and perform i2i communication, **characterized in that**
the aLP (102) is configured to monitor for a pacemaker mediated tachycardia (PMT) based on intrinsic atrial events detected by the aLP (102) and i2i communications from the vLP (104) indicating ventricular paced events;
the aLP (102) is configured to, in response to detection of compromised i2i communication from the vLP (104) to the aLP (102), transmit an i2i communication to the vLP (104) indicating compromised i2i communication from the vLP (104) to the aLP (102);
the aLP (102) is configured to interrupt its monitoring for PMT until i2i communication from the vLP (104) to the aLP (102) is functional; and
the vLP (104) is configured to, in response to reception of the i2i communication from the aLP (102) indicating compromised i2i communication from the vLP (104) to the aLP (102), monitor for a PMT based on ventricular paced events caused by the vLP (104) and i2i communications from the aLP (102) indicating atrial sensed events.

13. The dual chamber leadless pacemaker system (100) according to claim 12, wherein:
the aLP (102) is configured to monitor for VP-AS intervals for a predefined number of consecutive cardiac cycles when a pacing rate exceeds a PMT detection rate, wherein each of the VP-AS intervals represents a duration from reception of an i2i communication from the vLP (104) indicating a ventricular paced event to sensing an intrinsic atrial event by the aLP (102); and
the aLP (102) is configured to detect a PMT based on the monitored VP-AS intervals when the pacing rate exceeds the PMT detection rate.

14. The dual chamber leadless pacemaker system (100) according to any one of claims 12 or 13, wherein:
the vLP (104) is configured to monitor for VP-AS intervals for a predefined number of consecutive cardiac cycles when a pacing rate exceeds a PMT detection rate, wherein each of the VP-AS intervals represents a duration from delivery of a ventricular pacing pulse to the ventricular cardiac chamber by the vLP (104) to reception of an i2i communication from the aLP (102) indicating an atrial sensed event; and
the vLP (104) is configured to detect a PMT based on the monitored VP-AS intervals when the pacing rate exceeds the PMT detection rate.

15. The dual chamber leadless pacemaker system (100) according to any one of claims 12 through 14, wherein:
the aLP (102) further comprises:
at least one receiver (120, 122) connected to the at least two electrodes (108) and configured to receive i2i communications from the vLP (104);
a sensing circuitry (132) connected to the at least two electrodes (108) and configured to sense intrinsic atrial events;
at least one pulse generator (116) connected to the at least two electrodes (108) and configured to generate pacing pulses and i2i communication pulses; and
a controller (112) connected to the at least one receiver (120, 122), the sensing circuitry (132) and the at least one pulse generator (116) and configured to control the at least one receiver (120, 122), the sensing circuitry (132) and the at least one pulse generator (116); and
the vLP (104) further comprises:
at least one receiver (120, 122) connected to the at least two electrodes (108) and configured to receive i2i communications from the aLP (102);
a sensing circuitry (132) connected to the at least two electrodes (108) and configured to sense intrinsic ventricular events;
at least one pulse generator (116) connected to the at least two electrodes (108) and configured to generate pacing pulses and i2i communication pulses; and
a controller (112) connected to the at least one receiver (120, 122), the sensing circuitry (132) and the at least one pulse generator (116) and configured to control the at least one receiver (120, 122), the sensing circuitry (132) and the at least one pulse generator (116).

## Patentansprüche

1. System (100) aus kabellosem Zweikammer-Herzschrittmacher, umfassend:
einen atrialen kabellosen Schrittmacher, aLP, (102), der dazu konfiguriert ist, in oder an einer atrialen Herzkammer implantiert zu werden und mindestens zwei Elektroden (108) umfasst, die dazu angeordnet sind, ein intrinsisches atriales Ereignis zu erfassen, die atriale Herzkammer zu schrittsteuern und Implantat-zu-Implantat-(i2i-)Kommunikation durchzuführen; und
einen ventrikulären kabellosen Schrittmacher, vLP, (104), der dazu konfiguriert ist, in oder an einer ventrikulären Herzkammer implantiert zu werden und mindestens zwei Elektroden (108) umfasst, die dazu angeordnet sind, ein intrinsisches ventrikuläres Ereignis zu erfassen, die ventrikuläre Herzkammer zu schrittzusteuern und i2i-Kommunikation durchzuführen, **dadurch gekennzeichnet, dass**
der vLP (104) dazu konfiguriert ist, als Reaktion auf einen Empfang einer i2i-Kommunikation von dem aLP (102), die eine kompromittierte i2i-Kommunikation von dem vLP (104) an den aLP (102) angibt, auf eine schrittmachervermittelte Tachykardie (PMT) zu überwachen;
der vLP (104) dazu konfiguriert ist, nach der Detektion einer PMT ein PMT-PV-Intervall als Reaktion auf den Empfang einer i2i-Kommunikation von dem aLP (102) einzuleiten, die ein atriales erfasstes Ereignis angibt, wobei das PMT-PV-Intervall kürzer als ein PV-Intervall ist, das der vLP (104) ansonsten zum ventrikulären Schrittsteuern verwenden würde, wenn keine PMT detektiert wäre; und
der vLP (104) dazu konfiguriert ist, als Reaktion darauf, dass das PMT-PV-Intervall verstreicht, ohne dass ein intrinsisches ventrikuläres Ereignis während des PMT-PV-Intervalls detektiert wird, einen ventrikulären Stimulationsimpuls an die ventrikuläre Herzkammer abzugeben.

2. Kabelloses Zweikammer-Herzschrittmacher-System (100) nach Anspruch 1, wobei:
der aLP (102) dazu konfiguriert ist, als Reaktion auf das Erfassen eines intrinsischen atrialen Ereignisses eine atriale Refraktärzeit einzuleiten;
der aLP (102) dazu konfiguriert ist, keine i2i-Kommunikation, die ein atriales erfasstes Ereignis angibt, an den vLP (104) zu übertragen, wenn der aLP (102) ein weiteres intrinsisches atriales Ereignis während der atrialen Refraktärzeit erfasst; der aLP (102) dazu konfiguriert ist, als Reaktion darauf, dass die atriale Refraktärzeit verstreicht, einen atrialen Warnzeitraum einzuleiten; und
der aLP (102) dazu konfiguriert ist, als Reaktion darauf, dass der atriale Warnzeitraum verstreicht, einen atrialen Stimulationsimpuls an die atriale Herzkammer abzugeben.

3. Kabelloses Zweikammer-Herzschrittmacher-System (100) nach Anspruch 2, wobei:
der aLP (102) dazu konfiguriert ist, eine i2i-Kommunikation an den vLP (104) zu übertragen, die die Abgabe des atrialen Stimulationsimpulses an die atriale Herzkammer angibt; und
der vLP (104) dazu konfiguriert ist, als Reaktion auf den Empfang der i2i-Kommunikation von dem aLP (102), die die Abgabe des atrialen Stimulationsimpulses an die atriale Herzkammer angibt, die Abgabe eines ventrikulären Stimulationsimpulses an die ventrikuläre Herzkammer für einen Herzzyklus zu hemmen.

4. Kabelloses Zweikammer-Herzschrittmacher-System (100) nach einem der Ansprüche 1 bis 3, wobei der vLP (104) dazu konfiguriert ist, im Anschluss an die Abgabe des ventrikulären Stimulationsimpulses an die ventrikuläre Herzkammer als Reaktion darauf, dass das PMT-PV-Intervall verstreicht, ohne dass ein intrinsisches ventrikuläres Ereignis während des PMT-PV-Intervalls detektiert wird, das PV-Intervall als Reaktion auf den Empfang einer i2i-Kommunikation von dem aLP (102) einzuleiten, die ein atriales erfasstes Ereignis oder ein atriales stimuliertes Ereignis angibt.

5. Kabelloses Zweikammer-Herzschrittmacher-System (100) nach einem der Ansprüche 1 bis 4, wobei:
der vLP (104) dazu konfiguriert ist, als Reaktion auf eine Detektion einer nachfolgenden PMT das PMT-PV-Intervall als Reaktion auf den Empfang einer i2i-Kommunikation von dem aLP (102) einzuleiten, die ein atriales erfasstes Ereignis angibt; der vLP (104) dazu konfiguriert ist, im Anschluss an die Abgabe des ventrikulären Stimulationsimpulses an die ventrikuläre Herzkammer als Reaktion darauf, dass das PMT-PV-Intervall verstreicht, ohne dass ein intrinsisches ventrikuläres Ereignis während des PMT-PV-Intervalls detektiert wird, eine Dauer des PMT-PV-Intervalls während der Überwachung auf eine weitere nachfolgende PMT allmählich zu erhöhen; und
der vLP (104) dazu konfiguriert ist, als Reaktion auf eine Detektion der weiteren nachfolgenden PMT eine Dauer eines PV-Intervalls einzustellen, das der vLP (104) ansonsten zur ventrikulären Stimulation verwendet, wenn detektiert wurde, dass die PMT nicht gleich dem PMT-PV-Intervall oder länger als dieses, sondern kürzer als das erhöhte PMT-PV-Intervall war, das verwendet wird, wenn die weitere nachfolgende PMT detektiert wird.

6. Kabelloses Zweikammer-Herzschrittmacher-System (100) nach einem der Ansprüche 1 bis 5, wobei:
der aLP (102) dazu konfiguriert ist, auf eine PMT zu überwachen, wenn i2i-Kommunikation von dem vLP (104) zu dem aLP (102) funktional ist;
der aLP (102) dazu konfiguriert ist, als Reaktion auf eine Detektion einer kompromittierten i2i-Kommunikation von dem vLP (104) an den aLP (102) eine i2i-Kommunikation an den vLP (104) zu übertragen, die eine kompromittierte i2i-Kommunikation von dem vLP (104) an den aLP (102) angibt; und
der aLP (102) dazu konfiguriert ist, seine Überwachung auf eine PMT zu unterbrechen, bis i2i-Kommunikation von dem vLP (104) zu dem aLP (102) funktional ist.

7. Kabelloses Zweikammer-Herzschrittmacher-System (100) nach einem der Ansprüche 1 bis 6, wobei:
der aLP (102) dazu konfiguriert ist, als Reaktion auf eine Detektion einer kompromittierten i2i-Kommunikation von dem vLP (104) zu dem aLP (102) von einem kollektiven Betrieb mit dem vLP (104) in einem DDD- oder einem DDD(R)-Stimulationsmodus auf einen VDD- oder einen VDD(R)-Stimulationsmodus umzuschalten; und der vLP (104) dazu konfiguriert ist, als Reaktion auf einen Empfang einer i2i-Kommunikation von dem aLP (102), die eine kompromittierte i2i-Kommunikation von dem vLP (104) zu dem aLP (102) angibt, von dem kollektiven Betreiben mit dem aLP (102) in dem DDD- oder dem DDD(R)-Stimulationsmodus auf den VDD- oder den VDD(R)-Stimulationsmodus umzuschalten.

8. Kabelloses Zweikammer-Herzschrittmacher-System (100) nach einem der Ansprüche 1 bis 7, wobei:
der aLP (102) dazu konfiguriert ist, als Reaktion auf das Erfassen eines intrinsischen atrialen Ereignisses eine atriale Refraktärzeit einzuleiten;
der aLP (102) dazu konfiguriert ist, keine i2i-Kommunikation, die ein atriales erfasstes Ereignis angibt, an den vLP (104) zu übertragen, wenn der aLP (102) ein weiteres intrinsisches atriales Ereignis während der atrialen Refraktärzeit erfasst; und
das PMT-PV-Intervall eine Dauer aufweist, die so ausgewählt ist, dass eine beliebige retrograde P-Welle, die durch den ventrikulären Stimulationsimpuls induziert wird, der durch den vLP (104) an die ventrikuläre Herzkammer als Reaktion darauf abgegeben wird, dass das PMT-PV-Intervall verstreicht, ohne dass ein intrinsisches ventrikuläres Ereignis während des PMT-PV-Intervalls detektiert wird, innerhalb der atrialen Refraktärzeit liegt.

9. Kabelloses Zweikammer-Herzschrittmacher-System (100) nach einem der Ansprüche 1 bis 8, wobei das das PMT-PV-Intervall eine Dauer aufweist, die so ausgewählt ist, dass ein Ende des PMT-PV-Intervalls auftritt, während ein retrograder Leitungsweg von der ventrikulären Herzkammer zu der atrialen Herzkammer refraktär ist.

10. Kabelloses Zweikammer-Herzschrittmacher-System (100) nach einem der Ansprüche 1 bis 9, wobei:
der vLP (104) dazu konfiguriert ist, auf VP-AS-Intervalle für eine vordefinierte Anzahl aufeinanderfolgender Herzzyklen zu überwachen, wenn eine Stimulationsrate eine PMT-Detektionsrate überschreitet, wobei jedes der VP-AS-Intervalle eine Dauer von der Abgabe eines ventrikulären Stimulationsimpulses an die ventrikuläre Herzkammer durch den vLP (104) bis zum Empfang einer i2i-Kommunikation von dem aLP (102) darstellt, die ein atriales erfasstes Ereignis angibt; und
der vLP (104) dazu konfiguriert ist, eine PMT basierend auf den überwachten VP-AS-Intervallen zu detektieren, wenn die Stimulationsrate die PMT-Detektionsrate überschreitet.

11. Kabelloses Zweikammer-Herzschrittmacher-System (100) nach einem der Ansprüche 1 bis 10, wobei:
der aLP (102) ferner umfasst:
mindestens einen Empfänger (120, 122), der mit den mindestens zwei Elektroden (108) verbunden und dazu konfiguriert ist, i2i-Kommunikation von dem vLP (104) zu empfangen;
eine Erfassungsschaltung (132), die mit den mindestens zwei Elektroden (108) verbunden und dazu konfiguriert ist, intrinsische atriale Ereignisse zu erfassen;
mindestens einen Impulsgenerator (116), der mit den mindestens zwei Elektroden (108) verbunden und dazu konfiguriert ist, Stimulationsimpulse und i2i-Kommunikationsimpulse zu erzeugen; und
eine Steuerung (112), die mit dem mindestens einen Empfänger, der Erfassungsschaltung und dem mindestens einen Impulsgenerator verbunden und dazu konfiguriert ist, den mindestens einen Empfänger, die Erfassungsschaltung und den mindestens einen Impulsgenerator zu steuern; und
der vLP (104) ferner umfasst:
mindestens einen Empfänger (120, 122), der mit den mindestens zwei Elektroden (108) verbunden und dazu konfiguriert ist, i2i-Kommunikation von dem aLP (102) zu empfangen;
eine Erfassungsschaltung (132), die mit den mindestens zwei Elektroden (108) verbunden und dazu konfiguriert ist, intrinsische ventrikuläre Ereignisse zu erfassen;
mindestens einen Impulsgenerator (116), der mit den mindestens zwei Elektroden (108) verbunden und dazu konfiguriert ist, Stimulationsimpulse und i2i-Kommunikationsimpulse zu erzeugen; und
eine Steuerung (112), die mit dem mindestens einen Empfänger (120, 122), der Erfassungsschaltung (132) und dem mindestens einen Impulsgenerator (116) verbunden und dazu konfiguriert ist, den mindestens einen Empfänger (120, 122), die Erfassungsschaltung (132) und den mindestens einen Impulsgenerator (116) zu steuern.

12. Kabelloses Zweikammer-Herzschrittmacher-System (100), umfassend:
einen atrialen kabellosen Schrittmacher, aLP (102), der dazu konfiguriert ist, in oder an einer atrialen Herzkammer implantiert zu werden und mindestens zwei Elektroden (108) umfasst, die dazu angeordnet sind, ein intrinsisches atriales Ereignis zu erfassen, die atriale Herzkammer zu schrittsteuern und Implantat-zu-Implantat-(i2i-)Kommunikation durchzuführen; und
einen ventrikulären kabellosen Schrittmacher, vLP, (104), der dazu konfiguriert ist, in oder an einer ventrikulären Herzkammer implantiert zu werden und mindestens zwei Elektroden (108) umfasst, die dazu angeordnet sind, ein intrinsisches ventrikuläres Ereignis zu erfassen, die ventrikuläre Herzkammer zu schrittzusteuern und i2i-Kommunikation durchzuführen, **dadurch gekennzeichnet, dass**
der aLP (102) dazu konfiguriert ist, auf eine schrittmachervermittelte Tachykardie (PMT) basierend auf intrinsischen atrialen Ereignissen, die durch den aLP (102) detektiert werden, und i2i-Kommunikation von dem vLP (104), die ventrikuläre stimulierte Ereignisse angibt, zu überwachen;
der aLP (102) dazu konfiguriert ist, als Reaktion auf eine Detektion einer kompromittierten i2i-Kommunikation von dem vLP (104) an den aLP (102) eine i2i-Kommunikation an den vLP (104) zu übertragen, die eine kompromittierte i2i-Kommunikation von dem vLP (104) an den aLP (102) angibt;
der aLP (102) dazu konfiguriert ist, seine Überwachung auf eine PMT zu unterbrechen, bis i2i-Kommunikation von dem vLP (104) zu dem aLP (102) funktional ist; und
der vLP (104) dazu konfiguriert ist, als Reaktion auf den Empfang der i2i-Kommunikation von dem aLP (102), die eine kompromittierte i2i-Kommunikation von dem vLP (104) an den aLP (102) angibt, auf eine PMT basierend auf ventrikulären stimulierten Ereignissen, die durch den vLP (104) verursacht werden, und i2i-Kommunikation von dem aLP (102), die atriale erfasste Ereignisse angibt, zu überwachen.

13. Kabelloses Zweikammer-Herzschrittmacher-System (100) nach Anspruch 12, wobei:
der aLP (102) dazu konfiguriert ist, auf VP-AS-Intervalle für eine vordefinierte Anzahl aufeinanderfolgender Herzzyklen zu überwachen, wenn eine Stimulationsrate eine PMT-Detektionsrate überschreitet, wobei jedes der VP-AS-Intervalle eine Dauer ab dem Empfang einer i2i-Kommunikation von dem vLP (104), die ein ventrikuläres stimuliertes Ereignis angibt, bis zu dem Erfassen eines intrinsischen atrialen Ereignisses durch den aLP (102) darstellt; und
der vLP (102) dazu konfiguriert ist, eine PMT basierend auf den überwachten VP-AS-Intervallen zu detektieren, wenn die Stimulationsrate die PMT-Detektionsrate überschreitet.

14. Kabelloses Zweikammer-Herzschrittmacher-System (100) nach einem der Ansprüche 12 oder 13, wobei:
der vLP (104) dazu konfiguriert ist, auf VP-AS-Intervalle für eine vordefinierte Anzahl aufeinanderfolgender Herzzyklen zu überwachen, wenn eine Stimulationsrate eine PMT-Detektionsrate überschreitet, wobei jedes der VP-AS-Intervalle eine Dauer von der Abgabe eines ventrikulären Stimulationsimpulses an die ventrikuläre Herzkammer durch den vLP (104) bis zum Empfang einer i2i-Kommunikation von dem aLP (102) darstellt, die ein atriales erfasstes Ereignis angibt; und
der vLP (104) dazu konfiguriert ist, eine PMT basierend auf den überwachten VP-AS-Intervallen zu detektieren, wenn die Stimulationsrate die PMT-Detektionsrate überschreitet.

15. Kabelloses Zweikammer-Herzschrittmacher-System (100) nach einem der Ansprüche 12 bis 14, wobei:
der aLP (102) ferner umfasst:
mindestens einen Empfänger (120, 122), der mit den mindestens zwei Elektroden (108) verbunden und dazu konfiguriert ist, i2i-Kommunikation von dem vLP (104) zu empfangen;
eine Erfassungsschaltung (132), die mit den mindestens zwei Elektroden (108) verbunden und dazu konfiguriert ist, intrinsische atriale Ereignisse zu erfassen;
mindestens einen Impulsgenerator (116), der mit den mindestens zwei Elektroden (108) verbunden und dazu konfiguriert ist, Stimulationsimpulse und i2i-Kommunikationsimpulse zu erzeugen; und
eine Steuerung (112), die mit dem mindestens einen Empfänger (120, 122), der Erfassungsschaltung (132) und dem mindestens einen Impulsgenerator (116) verbunden und dazu konfiguriert ist, den mindestens einen Empfänger (120, 122), die Erfassungsschaltung (132) und den mindestens einen Impulsgenerator (116) zu steuern; und der vLP (104) ferner umfasst:
mindestens einen Empfänger (120, 122), der mit den mindestens zwei Elektroden (108) verbunden und dazu konfiguriert ist, i2i-Kommunikation von dem aLP (102) zu empfangen;
eine Erfassungsschaltung (132), die mit den mindestens zwei Elektroden (108) verbunden und dazu konfiguriert ist, intrinsische ventrikuläre Ereignisse zu erfassen;
mindestens einen Impulsgenerator (116), der mit den mindestens zwei Elektroden (108) verbunden und dazu konfiguriert ist, Stimulationsimpulse und i2i-Kommunikationsimpulse zu erzeugen; und
eine Steuerung (112), die mit dem mindestens einen Empfänger (120, 122), der Erfassungsschaltung (132) und dem mindestens einen Impulsgenerator (116) verbunden und dazu konfiguriert ist, den mindestens einen Empfänger (120, 122), die Erfassungsschaltung (132) und den mindestens einen Impulsgenerator (116) zu steuern.

## Revendications

1. Système de stimulateur cardiaque sans fil à double chambre (100) comprenant :
un stimulateur cardiaque sans fil auriculaire, aLP, (102) configuré pour être implanté dans une cavité cardiaque auriculaire ou sur celle-ci et comprenant au moins deux électrodes (108) agencées pour détecter un événement auriculaire intrinsèque, stimuler la cavité cardiaque auriculaire et réaliser une communication d'implant à implant (i2i) ; et
un stimulateur cardiaque sans fil ventriculaire, vLP, (104) configuré pour être implanté dans ou sur une cavité cardiaque ventriculaire et comprenant au moins deux électrodes (108) agencées pour détecter un événement ventriculaire intrinsèque, stimuler la cavité cardiaque ventriculaire et réaliser une communication i2i, **caractérisé en ce que**
le vLP (104) est configuré pour surveiller une tachycardie induite par un stimulateur cardiaque (PMT) en réponse à la réception d'une communication i2i provenant de l'aLP (102) indiquant une communication i2i compromise du vLP (104) vers l'aLP (102) ;
le vLP (104) est configuré pour, à la suite de la détection d'une PMT, lancer un intervalle PV de PMT en réponse à la réception d'une communication i2i provenant de l'aLP (102) indiquant un événement auriculaire détecté, dans lequel l'intervalle PV de PMT est plus court qu'un intervalle PV que le vLP (104) utiliserait autrement pour une stimulation ventriculaire si la PMT n'était pas détectée ; et
le vLP (104) est configuré pour administrer une impulsion de stimulation ventriculaire à la cavité cardiaque ventriculaire en réponse à l'expiration de l'intervalle PV de PMT sans qu'un événement ventriculaire intrinsèque n'ait été détecté pendant l'intervalle PV de PMT.

2. Système de stimulateur cardiaque sans fil à double chambre (100) selon la revendication 1, dans lequel :
l'aLP (102) est configuré pour lancer une période réfractaire auriculaire en réponse à la détection d'un événement auriculaire intrinsèque ;
l'aLP (102) est configuré pour ne transmettre aucune communication i2i indiquant un événement auriculaire détecté au vLP (104) si l'aLP (102) détecte un autre événement auriculaire intrinsèque pendant la période réfractaire auriculaire ;
l'aLP (102) est configuré pour lancer une période d'alerte auriculaire en réponse à l'expiration de la période réfractaire auriculaire ; et
l'aLP (102) est configuré pour administrer une impulsion de stimulation auriculaire à la cavité cardiaque auriculaire en réponse à l'expiration de la période d'alerte auriculaire.

3. Système de stimulateur cardiaque sans fil à double chambre (100) selon la revendication 2, dans lequel :
l'aLP (102) est configuré pour transmettre une communication i2i au vLP (104) indiquant l'administration de l'impulsion de stimulation auriculaire à la cavité cardiaque auriculaire ; et le vLP (104) est configuré pour, en réponse à la réception de la communication i2i provenant de l'aLP (102) indiquant l'administration de l'impulsion de stimulation auriculaire à la cavité cardiaque auriculaire, inhiber l'administration d'une impulsion de stimulation ventriculaire à la cavité cardiaque ventriculaire pour un cycle cardiaque.

4. Système de stimulateur cardiaque sans fil à double chambre (100) selon l'une quelconque des revendications 1 à travers 3, dans lequel le vLP (104) est configuré pour, à la suite de l'administration de l'impulsion de stimulation ventriculaire à la cavité cardiaque ventriculaire en réponse à l'expiration de l'intervalle PV de PMT sans qu'un événement ventriculaire intrinsèque n'ait été détecté pendant l'intervalle PV de PMT, lancer l'intervalle PV en réponse à la réception d'une communication i2i provenant de l'aLP (102) indiquant un événement auriculaire détecté ou un événement auriculaire stimulé.

5. Système de stimulateur cardiaque sans fil à double chambre (100) selon l'une quelconque des revendications 1 à travers 4, dans lequel :
le vLP (104) est configuré pour, en réponse à la détection d'une PMT ultérieure, lancer l'intervalle PV de PMT en réponse à la réception d'une communication i2i provenant de l'aLP (102) indiquant un événement auriculaire détecté ;
le vLP (104) est configuré pour, à la suite de l'administration de l'impulsion de stimulation ventriculaire à la cavité cardiaque ventriculaire en réponse à l'expiration de l'intervalle PV de PMT sans qu'un événement ventriculaire intrinsèque n'ait été détecté pendant l'intervalle PV de PMT, augmenter progressivement une durée de l'intervalle PV de PMT tout en surveillant une autre PMT ultérieure ; et
le vLP (104) est configuré pour, en réponse à la détection de l'autre PMT ultérieure, définir une durée d'un intervalle PV que le vLP (104) utilise autrement pour une stimulation ventriculaire si la PMT n'était pas détectée pour être égale à ou plus longue que l'intervalle PV de PMT mais plus courte que l'intervalle PV de PMT augmenté utilisé lorsque l'autre PMT ultérieure est détectée.

6. Système de stimulateur cardiaque sans fil à double chambre (100) selon l'une quelconque des revendications 1 à travers 5, dans lequel :
l'aLP (102) est configuré pour surveiller une PMT lorsque la communication i2i du vLP (104) vers l'aLP (102) est fonctionnelle ;
l'aLP (102) est configuré pour, en réponse à la détection d'une communication i2i compromise du vLP (104) vers l'aLP (102), transmettre une communication i2i au vLP (104) indiquant une communication i2i compromise du vLP (104) vers l'aLP (102) ; et l'aLP (102) est configuré pour interrompre sa surveillance d'une PMT jusqu'à ce que la communication i2i du vLP (104) vers l'aLP (102) soit fonctionnelle.

7. Système de stimulateur cardiaque sans fil à double chambre (100) selon l'une quelconque des revendications 1 à travers 6, dans lequel :
l'aLP (102) est configuré pour commuter d'un fonctionnement collectivement avec le vLP (104) dans un mode de stimulation DDD ou un mode de stimulation DDD(R) à un mode de stimulation VDD ou un mode de stimulation VDD(R) en réponse à la détection d'une communication i2i compromise du vLP (104) vers l'aLP (102) ; et le vLP (104) est configuré pour commuter d'un fonctionnement collectivement avec l'aLP (102) dans le mode de stimulation DDD ou le mode de stimulation DDD(R) au mode de stimulation VDD ou au mode de stimulation VDD(R) en réponse à la réception d'une communication i2i provenant de l'aLP (102) indiquant une communication i2i compromise du vLP (104) vers l'aLP (102).

8. Système de stimulateur cardiaque sans fil à double chambre (100) selon l'une quelconque des revendications 1 à travers 7, dans lequel :
l'aLP (102) est configuré pour lancer une période réfractaire auriculaire en réponse à la détection d'un événement auriculaire intrinsèque ;
l'aLP (102) est configuré pour ne transmettre aucune communication i2i indiquant un événement auriculaire détecté au vLP (104) si l'aLP (102) détecte un autre événement auriculaire intrinsèque pendant la période réfractaire auriculaire ; et l'intervalle PV de PMT a une durée sélectionnée de sorte que toute onde P rétrograde, induite par l'impulsion de stimulation ventriculaire administrée par le vLP (104) à la cavité cardiaque ventriculaire en réponse à l'expiration de l'intervalle PV de PMT sans qu'un événement ventriculaire intrinsèque n'ait été détecté pendant l'intervalle PV de PMT, tombe dans la période réfractaire auriculaire.

9. Système de stimulateur cardiaque sans fil à double chambre (100) selon l'une quelconque des revendications 1 à travers 8, dans lequel l'intervalle PV de PMT a une durée sélectionnée de sorte qu'une fin de l'intervalle PV de PMT survienne pendant qu'une voie de conduction rétrograde allant de la cavité cardiaque ventriculaire à la cavité cardiaque auriculaire est en période réfractaire.

10. Système de stimulateur cardiaque sans fil à double chambre (100) selon l'une quelconque des revendications 1 à travers 9, dans lequel :
le vLP (104) est configuré pour surveiller des intervalles VP-AS pour un nombre prédéfini de cycles cardiaques consécutifs lorsqu'une fréquence de stimulation dépasse une fréquence de détection de PMT, dans lequel chacun des intervalles VP-AS représente une durée allant de l'administration d'une impulsion de stimulation ventriculaire à la cavité cardiaque ventriculaire par le vLP (104) jusqu'à la réception d'une communication i2i provenant de l'aLP (102) indiquant un événement auriculaire détecté ; et
le vLP (104) est configuré pour détecter une PMT sur la base des intervalles VP-AS surveillés lorsque la fréquence de stimulation dépasse la fréquence de détection de PMT.

11. Système de stimulateur cardiaque sans fil à double chambre (100) selon l'une quelconque des revendications 1 à travers 10, dans lequel :
l'aLP (102) comprend en outre :
au moins un récepteur (120, 122) relié aux au moins deux électrodes (108) et configuré pour recevoir des communications i2i provenant du vLP (104) ;
un circuit de détection (132) relié aux au moins deux électrodes (108) et configuré pour détecter des événements auriculaires intrinsèques ;
au moins un générateur d'impulsions (116) relié aux au moins deux électrodes (108) et configuré pour générer des impulsions de stimulation et des impulsions de communication i2i ; et
un dispositif de commande (112) relié à l'au moins un récepteur, au circuit de détection et à l'au moins un générateur d'impulsions et configuré pour commander l'au moins un récepteur, le circuit de détection et l'au moins un générateur d'impulsions ; et
le vLP (104) comprend en outre :
au moins un récepteur (120, 122) relié aux au moins deux électrodes (108) et configuré pour recevoir des communications i2i provenant de l'aLP (102) ;
un circuit de détection (132) relié aux au moins deux électrodes (108) et configuré pour détecter des événements ventriculaires intrinsèques ;
au moins un générateur d'impulsions (116) relié aux au moins deux électrodes (108) et configuré pour générer des impulsions de stimulation et des impulsions de communication i2i ; et
un dispositif de commande (112) relié à l'au moins un récepteur (120, 122), au circuit de détection (132) et à l'au moins un générateur d'impulsions (116) et configuré pour commander l'au moins un récepteur (120, 122), le circuit de détection (132) et l'au moins un générateur d'impulsions (116).

12. Système de stimulateur cardiaque sans fil à double chambre (100) comprenant :
un stimulateur cardiaque sans fil auriculaire, aLP (102), configuré pour être implanté dans une cavité cardiaque auriculaire ou sur celle-ci et comprenant au moins deux électrodes (108) agencées pour détecter un événement auriculaire intrinsèque, stimuler la cavité cardiaque auriculaire et réaliser une communication d'implant à implant (i2i) ; et
un stimulateur cardiaque sans fil ventriculaire, vLP, (104) configuré pour être implanté dans une cavité cardiaque ventriculaire ou sur celle-ci et comprenant au moins deux électrodes (108) agencées pour détecter un événement ventriculaire intrinsèque, stimuler la cavité cardiaque ventriculaire et réaliser une communication i2i, **caractérisé en ce que**
l'aLP (102) est configuré pour surveiller une tachycardie induite par un stimulateur cardiaque (PMT) sur la base d'événements auriculaires intrinsèques détectés par l'aLP (102) et de communications i2i provenant du vLP (104) indiquant des événements ventriculaires stimulés ;
l'aLP (102) est configuré pour, en réponse à la détection d'une communication i2i compromise du vLP (104) vers l'aLP (102), transmettre une communication i2i au vLP (104) indiquant une communication i2i compromise du vLP (104) vers l'aLP (102) ;
l'aLP (102) est configuré pour interrompre sa surveillance d'une PMT jusqu'à ce que la communication i2i du vLP (104) vers l'aLP (102) soit fonctionnelle ; et
le vLP (104) est configuré pour, en réponse à la réception de la communication i2i provenant de l'aLP (102) indiquant une communication i2i compromise du vLP (104) vers l'aLP (102), surveiller une PMT sur la base d'événements ventriculaires stimulés provoqués par le vLP (104) et de communications i2i provenant de l'aLP (102) indiquant des événements auriculaires détectés.

13. Système de stimulateur cardiaque sans fil à double chambre (100) selon la revendication 12, dans lequel :
l'aLP (102) est configuré pour surveiller des intervalles VP-AS pour un nombre prédéfini de cycles cardiaques consécutifs lorsqu'une fréquence de stimulation dépasse une fréquence de détection de PMT, dans lequel chacun des intervalles VP-AS représente une durée allant de la réception d'une communication i2i provenant du vLP (104) indiquant un événement ventriculaire stimulé jusqu'à la détection d'un événement auriculaire intrinsèque par l'aLP (102) ; et
l'aLP (102) est configuré pour détecter une PMT sur la base des intervalles VP-AS surveillés lorsque la fréquence de stimulation dépasse la fréquence de détection de PMT.

14. Système de stimulateur cardiaque sans fil à double chambre (100) selon l'une quelconque des revendications 12 ou 13, dans lequel :
le vLP (104) est configuré pour surveiller des intervalles VP-AS pour un nombre prédéfini de cycles cardiaques consécutifs lorsqu'une fréquence de stimulation dépasse une fréquence de détection de PMT, dans lequel chacun des intervalles VP-AS représente une durée allant de l'administration d'une impulsion de stimulation ventriculaire à la cavité cardiaque ventriculaire par le vLP (104) jusqu'à la réception d'une communication i2i provenant de l'aLP (102) indiquant un événement auriculaire détecté ; et
le vLP (104) est configuré pour détecter une PMT sur la base des intervalles VP-AS surveillés lorsque la fréquence de stimulation dépasse la fréquence de détection de PMT.

15. Système de stimulateur cardiaque sans fil à double chambre (100) selon l'une quelconque des revendications 12 à travers 14, dans lequel :
l'aLP (102) comprend en outre :
au moins un récepteur (120, 122) relié aux au moins deux électrodes (108) et configuré pour recevoir des communications i2i provenant du vLP (104) ;
un circuit de détection (132) relié aux au moins deux électrodes (108) et configuré pour détecter des événements auriculaires intrinsèques ;
au moins un générateur d'impulsions (116) relié aux au moins deux électrodes (108) et configuré pour générer des impulsions de stimulation et des impulsions de communication i2i ; et
un dispositif de commande (112) relié à l'au moins un récepteur (120, 122), au circuit de détection (132) et à l'au moins un générateur d'impulsions (116) et configuré pour commander l'au moins un récepteur (120, 122), le circuit de détection (132) et l'au moins un générateur d'impulsions (116) ; et
le vLP (104) comprend en outre :
au moins un récepteur (120, 122) relié aux au moins deux électrodes (108) et configuré pour recevoir des communications i2i provenant de l'aLP (102) ;
un circuit de détection (132) relié aux au moins deux électrodes (108) et configuré pour détecter des événements ventriculaires intrinsèques ;
au moins un générateur d'impulsions (116) relié aux au moins deux électrodes (108) et configuré pour générer des impulsions de stimulation et des impulsions de communication i2i ; et
un dispositif de commande (112) relié à l'au moins un récepteur (120, 122), au circuit de détection (132) et à l'au moins un générateur d'impulsions (116) et configuré pour commander l'au moins un récepteur (120, 122), le circuit de détection (132) et l'au moins un générateur d'impulsions (116).
